# EUROPEAN PATENT SPECIFICATION

(11) **EP 4 268 595 B1**
(45) Date of publication and mention of the grant of the patent: **15.04.2026**
(21) Application number: 22170855.5
(22) Date of filing: 29.04.2022
(51) Int. Cl.: A01N 63/20, A01N 63/22, A01N 63/32, A01P 1/00, C12N 1/18, C12N 1/20

(54) **MICROBIAL COMPOSITIONS**
MIKROBIELLE ZUSAMMENSETZUNGEN
COMPOSITIONS MICROBIENNES

(43) Date of publication of application: 01.11.2023
(73) Proprietor: Multikraft Holding GmbH, 4632 Pichl bei Wels (AT)
(72) Inventor: HADER, Lukas, 4632 Pichl bei Wels (AT)
(74) Representative: Grünecker Patent- und Rechtsanwälte PartG mbB

(56) References cited:
- EP-A1- 3 909 589
- WO-A1-2019/133923
- WO-A2-2022/079261
- KR-A- 20030 020 766
- US-A1- 2020 131 096
- CANPOLAT ELIF ET AL: "Antifungal Activity of Some Lactic Acid Bacteria Against Several Soil-borne Fungal Pathogens Isolated from Strawberry Plants", TURKISH JOURNAL OF AGRICULTURE : FOOD SCIENCE AND TECHNOLOGY, vol. 6, no. 9, 15 September 2018 (2018-09-15), pages 1163 - 1167, XP093231535, ISSN: 2148-127X, Retrieved from the Internet <URL:http://agrifoodscience.com/index.php/TURJAF/article/download/1975/927> DOI: 10.24925/turjaf.v6i9.1163-1167.1975
- BAFFONI LOREDANA ET AL: "Microbial inoculants for the biocontrol of Fusarium spp. in durum wheat", vol. 15, no. 1, 1 December 2015 (2015-12-01), XP055893999, Retrieved from the Internet <URL:https://bmcmicrobiol.biomedcentral.com/track/pdf/10.1186/s12866-015-0573-7.pdf> DOI: 10.1186/s12866-015-0573-7
- VISSER R ET AL: "Antagonism of Lactic Acid Bacteria against Phytopathogenic Bacteria", APPLIED AND ENVIRONMENTAL MICROBIOLOGY, AMERICAN SOCIETY FOR MICROBIOLOGY, US, vol. 52, no. 3, 1 September 1986 (1986-09-01), pages 552 - 555, XP002992024, ISSN: 0099-2240

## Description

### Field of the Invention

The present invention relates to microbial compositions and methods useful in the field of agriculture for improving or maintaining plant health and for protecting plants from pathogens.

### Introduction

Conventional agricultural methods often rely on synthetic chemical plant protection products to prevent or treat infection of plants with plant pathogens. The use of these synthetic chemical plant protection products is often hazardous to the user and the biosphere and may have a negative impact on biodiversity. Often, the application of these traditional plant protection products deplete plant available nutrients in the soil and hence render crop plants even more susceptible to plant pathogens. In order to maintain high crop yields, ever increasing amounts of synthetic chemical plant protection products are required. Therefore, alternatives to synthetic chemical plant protection products have been long sought after. In particular, biopesticides based on beneficial microorganisms (bacteria, fungi, viruses and others), potentially containing further natural products, are being developed, to treat infections with plant pathogens, without the negative consequences outlined above. Biopesticides are typically produced by fermenting naturally occurring microorganisms and natural products.

Despite the advances in understanding the effects of certain beneficial microorganisms on soil quality and plant health, it has often been shown that the application of certain species of microorganisms have only a limited utility spectrum or are only useful for a particular soil/plant combination.

Baffoni L. et al. (BMC Microbiology, vol. 15, no. 1, December 2015) relates to microbial inoculants for the biocontrol of *Fusarium spp.* in durum wheat. Visser R. et al. (Appl. Env. Microbiol, vol. 52, no. 3, Sept. 1986, p. 552-5) relates to the antagonism of lactic acid bacteria against phytopathogenic bacteria. WO 2022/079261 A2 relates to a combination of *L. casei* with other Lactobacillus bacteria, including L. fermentum and L. plantarum, as well as with S. *cerevisiae* and *R. palustris,* for improving plant health, resistance to plant pathogens and reducing mycotoxin contamination of plant material. WO 2019/133923 relates to a microbial composition comprising at least one species of lactic acid bacteria, one Bacillus species and at least one yeast. US 2020/131096 relates to biofertiliser compositions containing, among others, *B. subtilis, L. casei, L. fermentum, L. plantarum, L. parafarraginis, L. rapi, Acetobacter ghanensis, Rhodopseudomonas palustris* and *S. cerevisiae.* KR 2003 0020766 relates to a biofertilizer composition comprising *R. palustris, L. plantarum, L. casei* and *S. cerevisiae.* EP3909589 relates to probiotic composition comprising *L. casei* and *L. plantarum.* Canpolat Elif et al. (Turkish Journal of Agriculture : Food Science and Technology, vol. 6, no. 9, Sept. 2018, p. 1163-67) relates to the antifungal activity of some lactic acid bacteria against several soil-borne fungal pathogens isolated from strawberry plants.

There remains a need for microbial compositions that can be used to treat and prevent a wide range of different plant pathogens, without the negative consequences of traditional synthetic chemical plant protection products. A further advantage of the microbial compositions of the present invention is that they can be applied in organic agriculture for the production of organic fruit and vegetables so that they meet the strict regulatory requirements for organic food production. The microbial compositions and method of the invention therefore help to meet the growing demand of organic food production.

### Summary of the Invention

In a first aspect, the present invention relates to the use of a microbial composition for treating or preventing an infection of a plant with *Fusarium poae.*

The microbial composition comprises *Lactobacillus casei, Lactobacillus plantarum, Lactobacillus fermentum, Saccharomyces cerevisiae, Rhodopseudomonas palustris* and *Acetobacter lovaniensis* as defined in the appended claims.

In a further aspect, the invention is directed to a method for preventing, treating, ameliorating or controlling an infection of a plant pathogen, the method comprising administering to the plant, to the seed of the plant, the soil the plant is rooted in, or otherwise exposing the plant, or the seed of the plant to an effective amount of the microbial composition, thereby treating the plant, preventing infection of the plant, ameliorating the infection of the plant or controlling the infection of the plant, wherein the infection is an infection with *Fusarium poae.*

In a further aspect, the invention is directed to a microbial composition comprising *Lactobacillus casei* DSMZ 20011, *Lactobacillus plantarum* DSMZ 21074, *Lactobacillus fermentum* DSMZ 20052, *Saccharomyces cerevisiae* DSMZ 70449, *Rhodopseudomonas palustris* ATCC 17001 and *Acetobacter lovaniensis* DSMZ 4491.

In an unclaimed aspect, the present disclosure is directed to a method of improving seed growth, plant growth, plant health and/or yield of plants or improve the plant's ability to grow on soils which are poor in available nutrients, the method comprising administering to the plant, to the seed of the plant, the soil the plant is rooted in, or otherwise exposing the plant, or the seed of the plant to an effective amount of the microbial composition. Yet another unclaimed aspect relates to a method of improving soil quality for plant growth.

The microbial composition comprises six of the microorganisms selected from the group consisting of *Lactobacillus casei, Lactobacillus plantarum, Lactobacillus fermentum, Saccharomyces cerevisiae, Rhodopseudomonas palustris* and *Acetobacter lovaniensis.*

The microbial composition comprises *Lactobacillus casei* DSMZ 20011.

The microbial composition comprises *Lactobacillus plantarum* DSMZ 21074.

The microbial composition comprises *Lactobacillus fermentum* DSMZ 20052.

The microbial composition comprises *Saccharomyces cerevisiae* DSMZ 70449.

The microbial composition comprises *Rhodopseudomonas palustris* ATCC 17001.

The microbial composition comprises *Acetobacter lovaniensis* DSMZ 4491.

The microbial composition, further comprising at least one microorganism selected from the group consisting of *Lactobacillus rhamnosus, Lactobacillus parafarraginis, Lactobacillus rapi, Lactobacillus zeae,* and *Lactobacillus harbinensis.*

The microbial composition, further comprising one, two, three, four, or five microorganisms selected from the group consisting *Lactobacillus rhamnosus, Lactobacillus parafarraginis, Lactobacillus rapi, Lactobacillus zeae,* and *Lactobacillus harbinensis.*

The microbial composition, further comprising *Lactobacillus rhamnosus, Lactobacillus parafarraginis, Lactobacillus rapi, Lactobacillus zeae,* and *Lactobacillus harbinensis.*

The microbial composition, wherein the composition comprises *Lactobacillus rhamnosus,* preferably *Lactobacillus rhamnosus* DSMZ 20021.

The microbial composition, wherein the composition comprises *Lactobacillus parafarraginis,* preferably *Lactobacillus parafarraginis* DSMZ 18390.

The microbial composition, wherein the composition comprises *Lactobacillus rapi,* preferably *Lactobacillus rapi* DSMZ 19907.

The microbial composition, wherein the composition comprises *Lactobacillus zeae,* preferably *Lactobacillus zeae* DSMZ 20178.

The microbial composition, wherein the composition comprises *Lactobacillus harbinensis.*

The microbial composition, further comprising at least one microorganism selected from the group consisting of *Bacillus subtilis, Bacillus amyloliquefaciens, Bacillus licheniformis,* and *Bacillus megaterium.*

The microbial composition, further comprising one, two, three, or four microorganisms selected from the group consisting of *Bacillus subtilis, Bacillus amyloliquefaciens, Bacillus licheniformis,* and *Bacillus megaterium.*

The microbial composition, further comprising *Bacillus subtilis, Bacillus amyloliquefaciens, Bacillus licheniformis,* and *Bacillus megaterium.*

The microbial composition, wherein the composition comprises *Bacillus subtilis,* preferably *Bacillus subtilis* DSMZ 10/149

The microbial composition, wherein the composition comprises *Bacillus amyloliquefaciens.*

The microbial composition, wherein the composition comprises *Bacillus licheniformis.*

The microbial composition, wherein the composition comprises *Bacillus megaterium.*

The microbial composition, further comprising at least one microorganism selected from the group consisting of *Lactobacillus buchneri, Lactobacillus diolivorans, Acetobacter indonesiensis, Pediococcus acidilactici, Acetobacter estunensis, Lactobacillus parabuchneri, Pseudomonas putida, Lactobacillus perolens,* and *Azotobacter vinelandii.*

The microbial composition, further comprising one, two, three, four, five, six, seven, eight, or nine microorganisms selected from the group consisting of *Lactobacillus buchneri, Lactobacillus diolivorans, Acetobacter indonesiensis, Pediococcus acidilactici, Acetobacter estunensis, Lactobacillus parabuchneri, Pseudomonas putida, Lactobacillus perolens,* and *Azotobacter vinelandii.*

The microbial composition, further comprising *Lactobacillus buchneri, Lactobacillus diolivorans, Acetobacter indonesiensis, Pediococcus acidilactici, Acetobacter estunensis, Lactobacillus parabuchneri, Pseudomonas putida, Lactobacillus perolens,* and *Azotobacter vinelandii.*

The microbial composition, wherein the composition comprises *Lactobacillus buchneri.*

The microbial composition, wherein the composition comprises *Lactobacillus diolivorans.*

The microbial composition, wherein the composition comprises *Acetobacter indonesiensis*

The microbial composition, wherein the composition comprises *Pediococcus acidilactici*

The microbial composition, wherein the composition comprises *Acetobacter estunensis*

The microbial composition, wherein the composition comprises *Lactobacillus parabuchneri.*

The microbial composition, wherein the composition comprises *Pseudomonas putida.*

The microbial composition, wherein the composition comprises *Lactobacillus perolens.*

The microbial composition, wherein the composition comprises *Azotobacter vinelandii.*

Exemplary microbial compositions/strain combinations combine all strains of Supplement 1 and may further include one or more (or all) strains of Supplement 2a, 2b, and/or Supplement 3 as defined in Table 1 below:

**Table 1**

| **Supplement 1 (Seed culture)** |
|---|
| *Lactobacillus casei* |
| *Lactobacillus plantarum* |
| *Lactobacillus fermentum* |
| *Saccharomyces cerevisiae* |
| *Rhodopseudomonas palustris* |
| *Acetobacter lovaniensis* |

| **Supplement 2a** |
|---|
| *Lactobacillus rhamnosus* |
| *Lactobacillus parafarraginis* |
| *Lactobacillus rapi* |
| *Lactobacillus zeae* |
| *Lactobacillus harbinensis* |

| **Supplement 2b** |
|---|
| *Bacillus subtilis* |
| *Bacillus amyloliquefaciens* |
| *Bacillus licheniformis* |
| *Bacillus megaterium* |

| **Supplement 3** |
|---|
| *Lactobacillus buchneri* |
| *Lactobacillus diolivorans* |
| *Acetobacter indonesiensis* |
| *Pediococcus acidilactici* |
| *Acetobacter estunensis* |
| *Lactobacillus parabuchneri* |
| *Pseudomonas putida* |
| *Lactobacillus perolens* |
| *Azotobacter vinelandii* |

Species of Supplement 2a, 2b and 3 have probiotic and/or antifungal properties and/or may be silage additives. Species of Supplement 3 such as *A. indonesiensis, A. estunensis* and A. *vinelandii* are capable of nitrogen fixation. Species of Supplement 3 such as *P. putida* are to be used if in addition to pathogen control also a fertilizing or soil activating effect is desired.

In some embodiments the species to be included in the microbial composition, in particular strains of these species, can be obtained from existing sources such as depository institutions (e.g. German Collection of Microorganisms and Cell Cultures GmbH, DSMZ or the American Type Culture Collection (ATCC)). Generally, for the sake of the present invention the species and its properties are important and not a particular strain of the species, i.e. all strains of a given species exhibit the necessary and required properties that characterize the present invention. While the selection of a particular strain of any of the species mentioned hereinabove is not required, one or more of the following deposited strains may be used:
*Lactobacillus casei* ATCC 393 T; BCRC 10697; CCRC 10697; CCUG 21451; CECT 475; CIP 103137; DSM 20011; Hucker03; IAM 12473; IFO 15883; KCTC 3109; LMG 6904; NBRC 15883; NCIB 11970; NCIMB 11970; NRRL B-1922; Orla-Jensen 7; OrlandL-323; Tittsler303, *Lactobacillus plantarum* ATCC 14917; CCUG 30503; CIP 103151; DSM 20174; IFO 15891; JCM 1149; LMG 6907; NBRC 15891; NCIB 11974; NCIMB 11974; NRRL B-4496; *Lactobacillus fermentum* ATCC 14931; CCUG 30138; CIP 102980; DSM 20052; IFO 15885; JCM 1173; LMG 6902; NBRC 15885; NCCB 46038; NCDO 1750; NCFB 1750; NCIB 11840; NCIMB 11840; NRRL B-4524; *Saccharomyces cerevisiae* ATCC 18824, CBS 1171, NCYC 505, NRRL Y-12632, JCM 7255, DSMZ 70449, DSM 1333, 1334, 1848, 2155, 3797, 3798, 3799, 3820, 4266, 4267, 70416, 70424, 70449, 70451 to 70469, 70471, 70478, 70487, 70509, 70514, 70523, 70524, 70868, 70869, 106003, 106004, 107176, and 107177, ISO0203; *Rhodopseudomonas palustris* 2.1.6; ATCC 17001; ATH 2.1.6; BCRC 16408; Pa16; *Acetobacter lovaniensis* ATCC 12875; DSM 4491; DSM 46218; IFO 13753; LMG 1579; LMG 1617; NBRC 13753; NCIB 8620; NCIMB 8620, *Lactobacillus rhamnosus* ATCC 7469; CCUG 21452; CIP A157; DSM 20021; IFO 3425; JCM 1136; LMG 6400; NBRC 3425; NCAIM B.01147; NCCB 46033; NCDO 243; NCFB 243; NCIB 6375; NCIMB 6375; NCTC 12953; NRRL B-442; VKM B-57; VKM B-574, *Lactobacillus parafarraginis* DSM 18390; JCM 14109; NRIC 677, *Lactobacillus rapi* DSM 19907; JCM 15042; NRIC 743; YIT 11204, *Lactobacillus zeae* ATCC 15820; BCRC 17942; CCUG 35515; DSM 20178; JCM 11302; LMG 17315; NCIB 9537; NCIMB 9537, *Bacillus subtilis* DSMZ 10/149, ATCC 6051; ATCC 6051-U; BCRC 10255; CCM 2216; CCRC 10255; CCUG 163; CCUG 163 B; CFBP 4228; CIP 52.65; DSM 10; IAM 12118; IFO 13719; IFO 16412; IMET 10758; JCM 1465; LMG 7135; NBRC 13719; NBRC 16412; NCAIM B.01095; NCCB 32009; NCCB 53016; NCCB 70064; NCDO 1769; NCFB 1769; NCIMB 3610; NCTC 3610; NRRL B-4219; NRRL NRS-1315; NRRL NRS-744; VKM B-501; *Lactobacillus harbinensis* AHU 1762; DSM 16991; JCM 16178; NBRC 100982; SBT 10908, *Bacillus amyloliquefaciens* ATCC 23350; BCRC 11601; CCUG 28519; CFBP 4246; CIP 103265; DSM 7; Fukumoto strain F; HAMBI 1824; IFO 15535; LMG 12234; LMG 9814; NBRC 15535; NCAIM B.01704; NCIMB 12077; NRRL B-14393, *Bacillus licheniformis* ATCC 14580; BCRC 11702; CCM 2145; CCRC 11702; CCUG 7422; CFBP 4227; CIP 52.71; DSM 13; HAMBI 1823; IAM 13417; IFO 12200; JCM 2505; KCTC 1753; KCTC 1918; LMG 12363; LMG 12407; LMG 6933; NBRC 12200; NCAIM B.01470; NCCB 50016; NCCB 75015; NCDO 1772; NCFB 1772; NCIB 9375; NCIMB 9375; NCTC 10341; NRRL NRS-1264; VKM B-511, *Bacillus megaterium* ATCC 14581; BCRC 10608; CCM 2007; CCRC 10608; CCUG 1817; CIP 66.20; DSM 32; HAMBI 2018; IAM 13418; IFO 15308; JCM 2506; KCTC 3007; LMG 7127; NBRC 15308; NCCB 75016; NCIB 9376; NCIMB 9376; NCTC 10342; NRIC 1710; NRRL B-14308; VKM B-512, *Lactobacillus buchneri* CCUG 21532; CIP 103023; DSM 20057; JCM 1115; LMG 6892; NCAIM B.01145; NRRL B-1837; VKM B-1599, *Lactobacillus diolivorans* DSM 14421; JCM 12183; JKD6; LMG 19667, *Acetobacter indonesiensis* 5H-1; DSM 15552; IFO 16471; JCM 10948; LMG 19824; NBRC 16471; NRIC 313, *Pediococcus acidilactici* B213c; Back S213C; CCUG 32235; CIP 103408; DSM 20284; LMG 11384; NCFB 2767; NCIMB 12174, *Acetobacter estunensis* ATCC 23753; DSM 4493; IFO 13751; JCM 21172; LMG 1626; NBRC 13751; NCIB 8935; NCIMB 8935, *Lactobacillus parabuchneri* 6E; ATCC 49374; CCUG 32261; CIP 103368; DSM 5707; JCM 12493; LMG 11457; NCDO 2748; NCFB 2748; NCIB 8838; NCIMB 8838, *Pseudomonas putida* ATCC 12633; CCUG 12690; CFBP 2066; CIP 52.191; DSM 291; DSM 7314; HAMBI 7; IFO 14164; JCM 13063; JCM 20120; LMG 2257; NBRC 14164; NCAIM B.01634; NCCB 68020; NCCB 72006; NCTC 10936, *Lactobacillus perolens* DSM 12744; JCM 12534; L 532; L 533 T; LMG 18936, *Azotobacter vinelandii* ATCC 478; DSM 2289; JCM 21475; LMG 8758; NBRC 102612; NCCB 26007; NRRL B-14641; VKM B-1617.

Strains of the microbial composition of the present invention are *Lactobacillus casei* DSMZ *20011, Lactobacillus plantarum* DSMZ 21074, *Lactobacillus fermentum* DSMZ 20052, *Saccharomyces cerevisiae* DSMZ 70449, *Rhodopseudomonas palustris* ATCC 17001, and *Acetobacter lovaniensis* DSMZ 4491.

The invention further relates to the use of the microbial composition for treating or preventing an infection of a plant with a plant pathogen as specified in the claims.

A method for treating or preventing an infection of a plant with a plant pathogen, the method comprising administering to the plant, the seed of the plant, the soil the plant is rooted in, or otherwise exposing the plant, or the seed of the plant to an effective amount of the microbial composition, thereby treating the plant or preventing infection of the plant as specified in the claims.

The plant pathogen is *Fusarium poae.*

In an unclaimed aspect the present disclosure relates to the microbial composition, the use, or the method, wherein the bacterial pathogen is selected from the group consisting of *Phytoplasma spp., Spiroplasma spp., Streptomyces scabies, Xanthomonas spp.,* such as *Xanthomonas campestris pv. Vesicatoria, Pseudomonas spp.,* such as *P*. *savastanoi, P. syringae, P. aeruginosa,* and *P*. *fluorescens, E. coli, Listeria monocytogenes, Staphylococcus spp.,* such as *S. aureus, Streptococcus spp.,* such as *S*. *uberis, Agrobacterium tumefaciens, Burkhoideria cepacia, Erwinia carotovora, Erwinia chrysanthemi, Pantoea agglomerons, Ralstonia solanacearum, Pantoea stewartii, Aeromonas hydrophila, Vibrio spp.,* such as *V. anguillarum, V. harveyi, V. cholera,* and *V. parahaemoliticus, Actinobacillus pleuropneumoniae, Chromobacter violaceum, Coxiella burnetti, Francisella tularensis, Haemophilus influenza, Pasteurella multocida, Shigella flexneri, Salmonella typhi, Salmonella typhimurium, Yersinia pestis,* and *Yersinia pseudotuberculosis.*

In an unclaimed aspect the present disclosure relates to the microbial composition, the use, or the method, wherein the fungal pathogen is selected from the group consisting of *Pestalopsis spp., Neopestalotiopsis spp., Fusarium spp.,* such as *F. avenaceum, F. culmorum, F. graminearum, F. oxysporum* and special forms thereof, including *F. oxysporum f. sp. zingiberi, ~ f. sp. niveum,* and ~ f. *sp, cubense, Collectotrichum coccodes, Phytophthora spp.,* such as *Phytophthora infestans, Phytophthora erythroseptica, Phytophthora cinnamomi, Rhizoctonia spp.,* such as *Rhizoctonia solani, Corynespora cassiicola, Puccinia spp.,* such as *Puctinia arachidus, Puccinia striiformis, Puccinia graminis f. sp. Tritici, Botrytis cinerea, Rhizoctonia, Pythium ultimum, Pythium myriotylum, Psuedocercospora macadamiae, Rosellinia necartrix, Verticillium spp.,* such as *Verticillium deliliae, Phialemonium dimorphosporum, Thielaviopsis spp., Magnaporthe grisea, Sclerotinia sclerotiorum, Ustilago spp., Phakospora pachyrhizi,* and *Armillaria spp.*

In an unclaimed aspect the present disclosure relates to the microbial composition, the use, or the method, wherein the viral pathogen is selected from the group consisting of Cucumovirus (e.g. *Cucumber mosaic virus*)*,* Potyvirus (e.g. *Lettuce mosaic virus, Plum pox virus*)*,* Tobamovirus (e.g. *Tomato mosaic virus*)*,* or Orthotospovirus (e.g. *Tomato spotted wilt orthotospovirus*)*.*

In an unclaimed aspect the present disclosure further relates to a method for increasing plant growth and/or productivity using the microbial compositions of the invention, the method comprising applying to the plant, plant seeds or to the soil in which the plant or plant seeds are grown an effective amount of a microbial composition as described hereinabove or below.

In an unclaimed aspect the present disclosure further relates to a method for improving soil quality using the microbial compositions of the invention, the method comprising applying to soil or to the plants or plant seeds in said soil an effective amount of a microbial composition as described hereinabove or below.

The microbial composition, the use, or the method of, wherein the plant is a pasture plant, an ornamental plant, or a crop plant, including a fruit, a vegetable, a nut, a seed or a grain.

### Description of the Figures

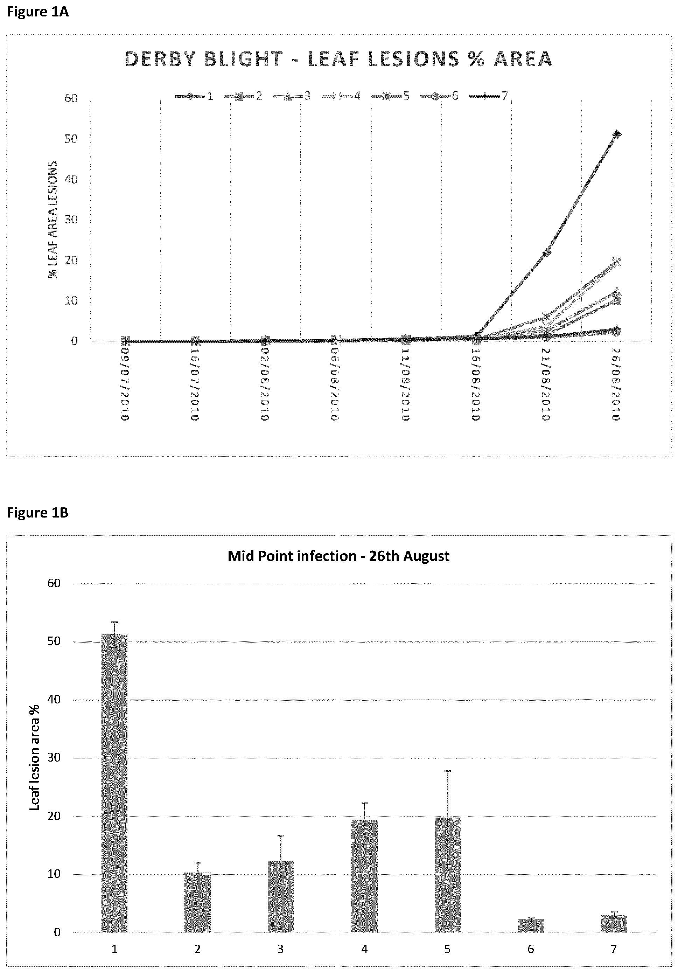

Figure 1: Late blight field trial in potatoes; 1 = Untreated control; 2 = MK5 3L/ha; 3 = Product 1 20L/ha + 3kg Ceramic powder; 4 = Product 1 10L/ha + 3kg Ceramic powder; 5 = *B. amyloliquefaciens* + wetter; 6 = Phosphite and chemical fungicide; 7 = Chemical fungicide. Fig. 1A: leaf lesion area in % at indicated time points; Fig. 1B: Leaf lesion area in % at mid-point infection.

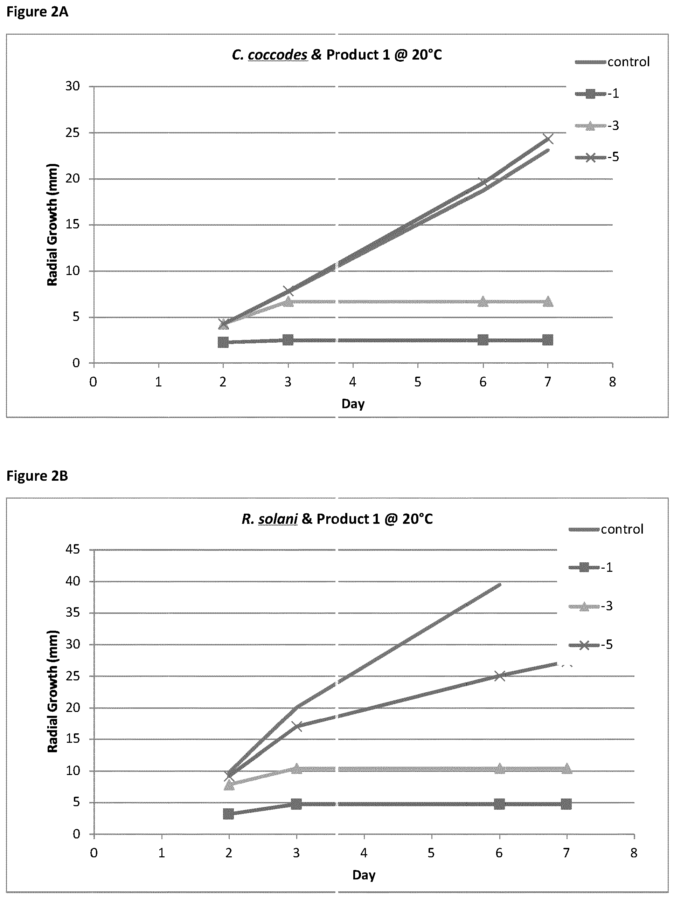

Figure 2: Inhibitory effect against *Colletotrichum coccodes* and *Rhizoctonia solani* (agar plate trial). Fig 2A, 2B legend: control = agar without Product 1, - 1 = 0.1%; -3 = 0.001%; -5 = 0.00001% of microbial composition (Product 1) in the agar. Fig. 2A: radial growth of C. *coccoides* at 20°C in the presence of different concentrations of microbial composition (Product 1). Fig. 2B: radial growth of *R. solani* at 20°C in the presence of different concentrations of microbial composition (Product 1).

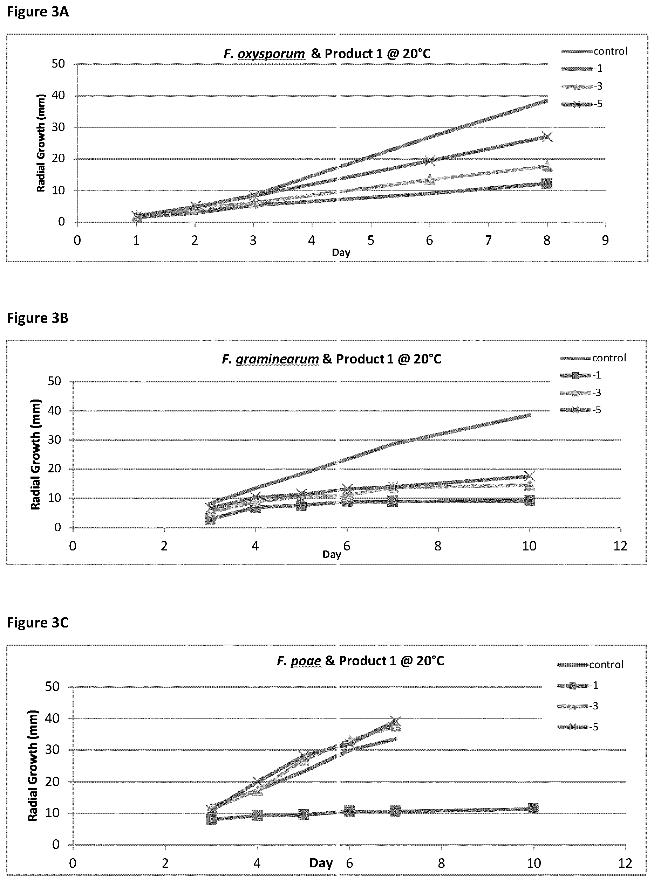

Figure 3: Inhibitory effect against *Fusarium graminearum, F. oxysporum, and F. poae* (agar plate trial). Fig 3A, 3B, 3C legend: control = agar without Product 1, - 1 = 0.1%; -3 = 0.001 %; -5 = 0.00001% of microbial composition (Product 1) in the agar. Fig. 3A: radial growth of *F*. *oxysporum* at 20°C in the presence of different concentrations of microbial composition (Product 1). Fig. 3B: radial growth of *F. graminearum* at 20°C in the presence of different concentrations of microbial composition (Product 1). Fig. 3C: radial growth of *F. poae* at 20°C in the presence of different concentrations of microbial composition (Product 1).

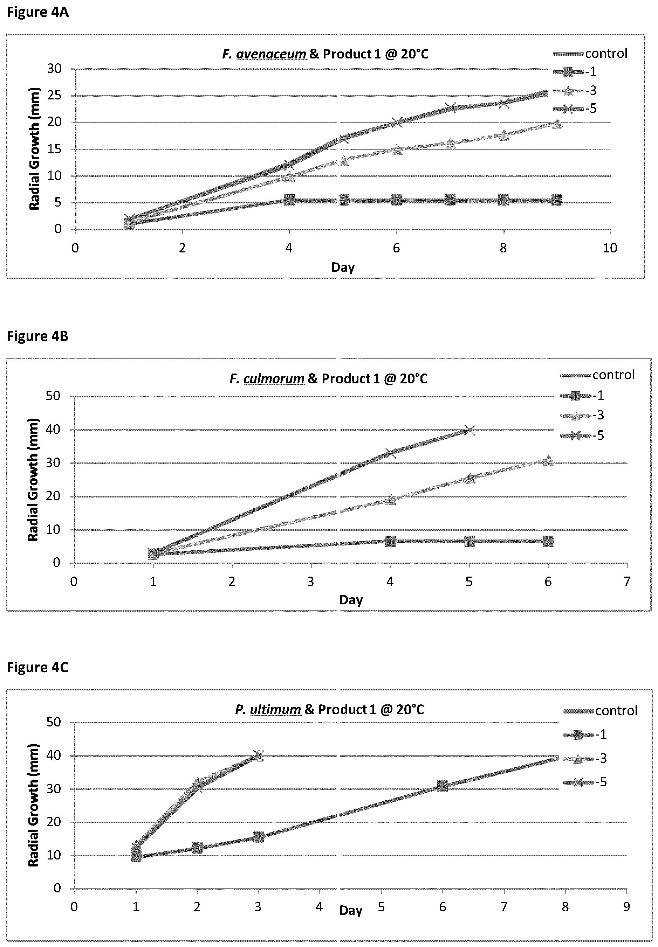

Figure 4: Inhibitory effect against *Fusarium avenaceum, F. culmorum, and Pythium ultimum* (agar plate trial). Fig 4A, 4B, 4C legend: control = agar without Product 1, - 1 = 0.1%; -3 = 0.001%; -5 = 0.00001% of microbial composition (Product 1) in the agar. Fig. 4A: radial growth of *F. avenaceum* at 20°C in the presence of different concentrations of microbial composition (Product 1). Fig. 4B: radial growth of *F. culmorum* at 20°C in the presence of different concentrations of microbial composition (Product 1). Fig. 4C: radial growth of *Pythium ultimum* at 20°C in the presence of different concentrations of microbial composition (Product 1).

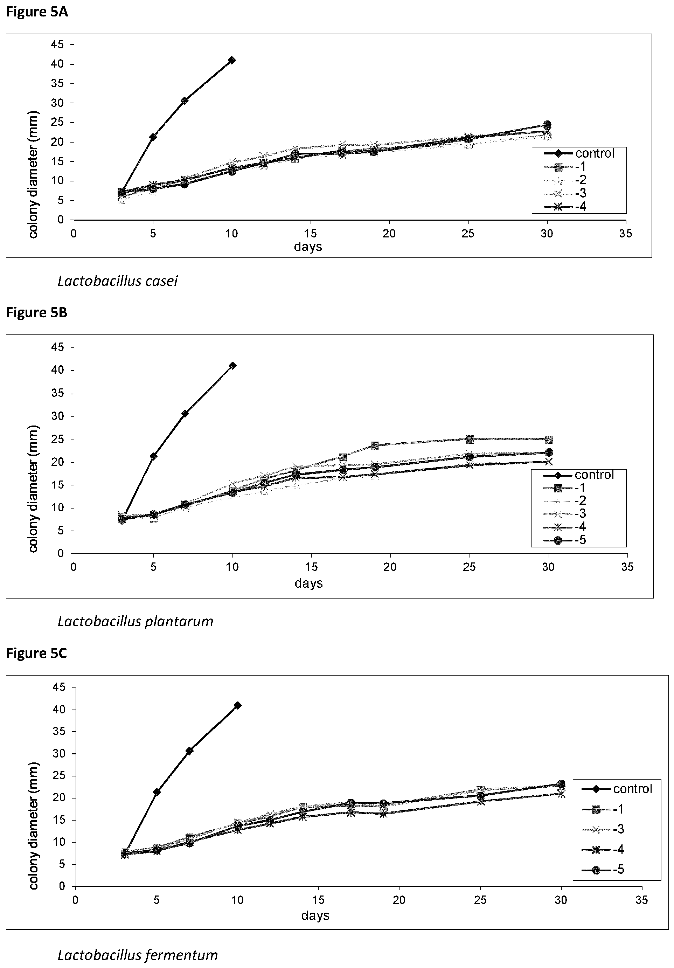

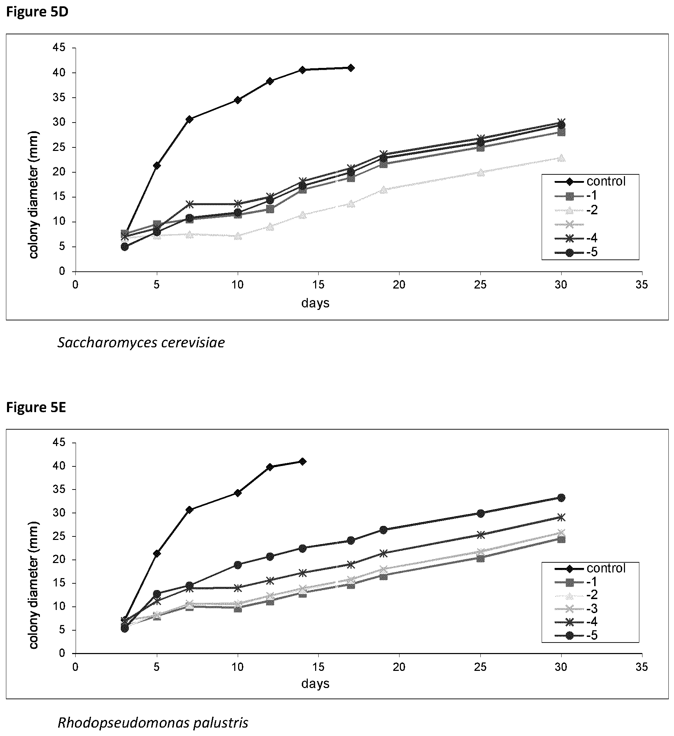

Figure 5: Inhibitory effect of *Lactobacillus casei, L. plantanarum, L. fermentum, Saccharomyces cerevisiae, and Rhodopseudomonas palustris* (agar plate trial). Fig 5A-E legend: control = PDA without lead strain, - 1 = dilution by 10; -2= dilution by 100, -3 = dilution by 1000; -4= dilution by 10000 -5 = dilution by 100000. Fig. 5A: colony diameter of pathogens in the presence of *L*. *casei.* Fig. 5B: colony diameter of pathogens in the presence of *L. plantanarum.* Fig. 5C: colony diameter of pathogens in the presence of *L. fermentum.* Fig. 5D: colony diameter of pathogens in the presence of *S. cerevisiae.* Fig. 5E: colony diameter of pathogens in the presence of *R*. *palustris.*

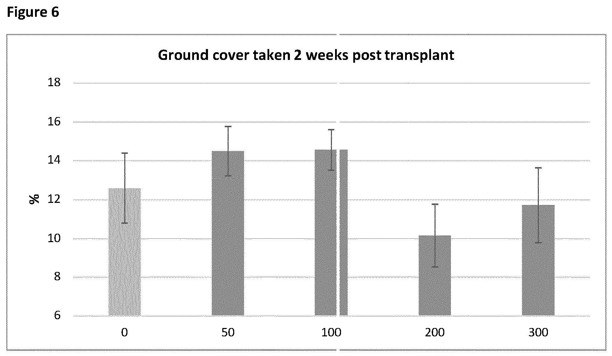

Figure 6: Ground cover of Kale transplants taken 2 weeks post-transplant.

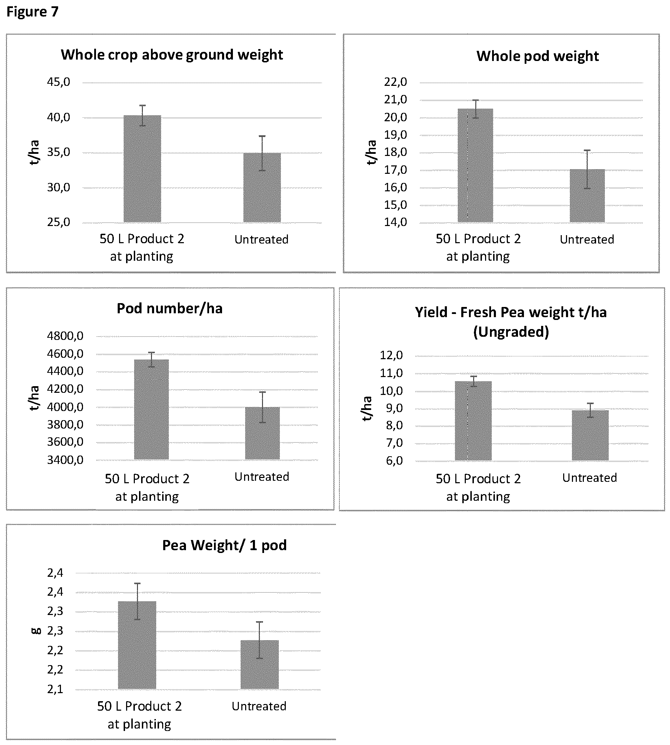

Figure 7: Yield data of pea (variety Oasis) of treated plots vs. untreated control.

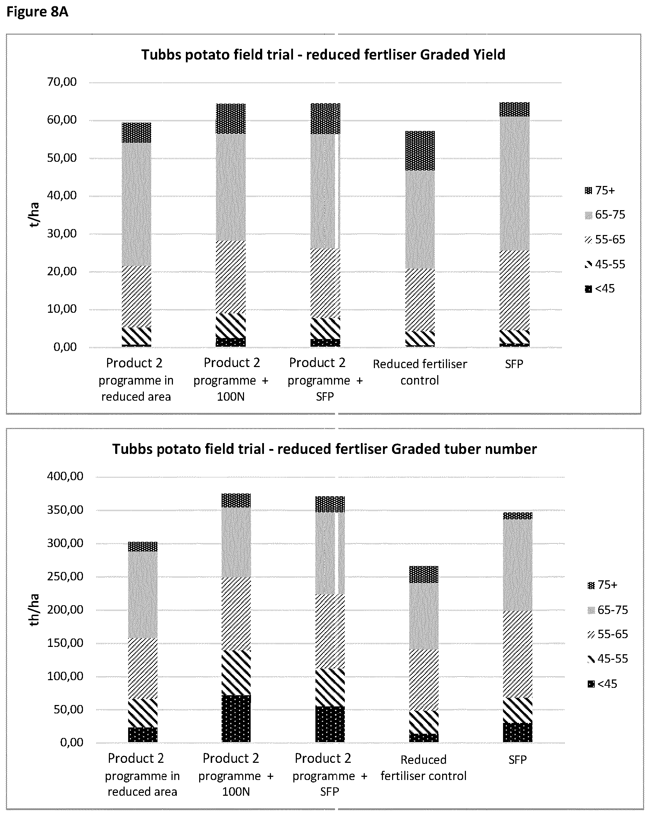

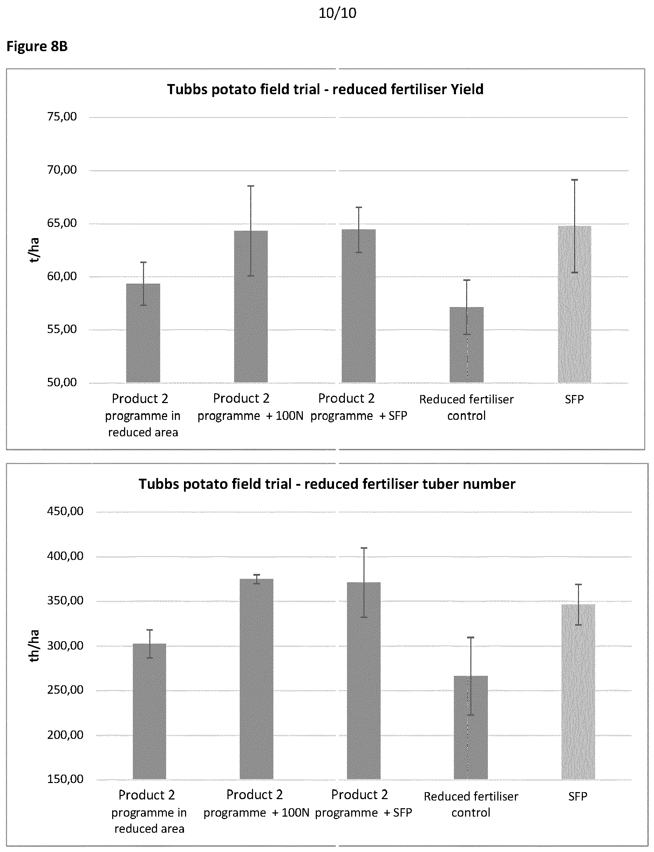

Figure 8: Field plot data of Tubbs potato field trial using different fertilizing treatments. Fig. 8A: size distribution and graded yield (top) and size distribution and graded tube number (bottom) for different soil/fertilizer treatments. Fig. 8B: overall yield (top) and tuber number (bottom) for different soil/fertilizer treatments.

### Detailed Description of the Invention

Unless defined otherwise, all technical and scientific terms used herein have the same meaning as commonly understood by those of ordinary skill in the art. Although any methods and materials similar or equivalent to those described herein can be used in the practice or testing of the present disclosure, typical methods and materials are described.

The use of the term "comprising" as well as other grammatical forms such as "comprises" and "comprised" is not limiting. The terms "comprising", "comprises" and "comprised" should be understood as referring to an open-ended description of an embodiment of the present invention that may, but does not have to, include additional technical features in addition to the explicitly stated technical features. In the same sense, the term "involving" as well as other respective grammatical forms such as "involves" and "involved" is not limiting. The same applies for the term "including" and other grammatical forms such as "includes" and "included". Section headings throughout the description are for organizational purposes only. In particular, they are not intended as limiting for various embodiments described therein, and it is to be understood that embodiments (and features therein) described under one subheading may be freely combined with embodiments (and features therein) described under another subheading. Further, the terms "comprising", "involving" and "including", and any grammatical forms thereof, are not to be interpreted to exclusively refer to embodiments that include additional features to those explicitly recited. These terms equally refer to embodiments that consist of only those features that are explicitly mentioned.

As used herein, the term "about" when referring to a measurable value such as an amount, a temporal duration, and the like, is meant to encompass, in addition to that measurable value itself, variations of ±20% or ±10%, in some instances ±9%, ±8%, ±7%, ±6%, ±5%, ±4%, ±3%, ±2% or ±1%, and in some instances ±0.9%,±0.8%, ±0.7%, ±0.6%, ±0.5%, ±0.4%, ±0.3%, ±0.2% or ±0.1% from the specified value. It is to be understood that the term "about", in reference to a particular value, includes that exact particular value itself, irrespective of any explicit mention that that exact particular value is included. Thus, the absence of an explicit indication that the term "about" includes the particular exact recited value is not to be understood that the particular recited value is excluded from the range of variations created by the term "about"; even in the absence of an explicit indication that the term "about" includes the particular exact recited value, that exact particular value is still included in said range of variations created by the term "about". The skilled person will recognize when deviations around a stated value may be either integral or fractional (as for example for a temperature or a pressure), and when such deviations may only be integral (as for example for discrete moieties within a larger molecule).

By "improving soil quality" is meant increasing the amount and/or availability of nutrients required by, or beneficial to plants, for growth. By way of example, such nutrients include nitrogen, phosphorous, potassium, copper, zinc, boron and molybdenum. Also encompassed by the term "improving soil quality" is reducing or minimising the amount of an element that may be detrimental to plant growth or development such as, for example iron and manganese. Thus, improving soil quality by use of microbial compositions of the present disclosure thereby assists and promotes the growth of plants in the soil. By improving the soil quality, the microbial compositions also prevent infection of the plant with plant pathogens.

The term "effective amount" refers to an amount of microbial composition applied to a plant, a plant seed, or a given area of soil or vegetation that is sufficient to effect one or more beneficial or desired outcomes, for example, in terms of plant growth rates, crop yields, nutrient availability in the soil, or in particular, treatment or prevention of a plant disease caused by a plant pathogen. An "effective amount" can be provided by administering the microbial composition once, twice, three times, four times, multiple times or intermittently. The duration between the administrations may be a few days, e.g., about 2, 3, 4, 5, 6, 7, 8, 9, 10, 11, 12, 13, 14, 15, 16, 17, 18, 19 or 20 days. The duration between the administrations may be a few weeks, e.g., about 2, 3, 4, 5, 6, 7, 8, 9, 10, 11, 12, 13, 14, 15, 16, 17, 18, 19 or 20 weeks.

A use herein the term "exposing" generally means bringing into contact with. Exposure of a plant, a plant seed, or a soil to the microbial composition includes administration of the microbial composition to the plant, the plant seed, or the soil or otherwise bringing the composition into contact with the plant, the plant seed, or the soil whether directly or indirectly. For example, exposing a plant to the microbial composition may include applying or administering the microbial composition to an environment inhabited by the plant or to a liquid or other nutrient composition to be administered to the plant. In the present disclosure the terms "exposing", "administering" and "contacting" and variations thereof may, in some contexts, be used interchangeably.

The term "inhibiting" and variations thereof such as "inhibition" and "inhibits" as used herein in relation to the plant pathogen refers to any activity of the microbial composition described herein that leads to a reduction of the growth of the pathogen, that kills the pathogen or reduction of any negative effects of the pathogen on plant health and growth. Such inhibition may be absolute or partial and/or may be temporal or spatial in nature. Inhibition of the growth of bacteria, fungi or virus by a composition can be assessed by measuring microbial growth in the presence and absence of the composition. The microbial growth may be inhibited by the composition by at least or about 10%, 15%, 20%, 25%, 30%, 35%, 40%, 45%, 50%, 55%, 60%, 65%, 70%, 75%, 80 % 85%, 90%, 95% or more compared to the growth of the same microbe that is not exposed to the composition.

The term "treating", "treatment" and the like refer to an and all applications which remedy, or otherwise hinder, retard, or reverse the progression of an infection or disease or at least one symptom of an infection or disease, including reducing the severity of an infection or disease. Thus, treatment dose no necessarily imply that a subject is treated until complete elimination of the infection or recovery from a disease. Similarly, the terms "preventing", "prevention" and the like refer to any and all applications which prevent the establishment of an infection or disease or otherwise delay the onset of an infection or disease or reduce the severity of an infection or disease as compared to if no prevention measures had been taken.

The term "plant productivity" as used herein refers to any aspect of growth or development of a plant, that is a reason for which the plant is grown. Thus, "improved" or "increased" plant productivity refers broadly to improvements (as compared to untreated plants) in biomass or yield of leaves, stems, grain, fruit, vegetables, flowers, or other plant parts harvested or used for various purposes, and improvements in growth of plant parts, including stems, leaves and roots. For example, when referring to food crops, such as grains, fruits or vegetables, plant productivity may refer to the yield of grain, fruit, vegetables or seeds harvested from a particular crop. For crops such as pasture, plant productivity may refer to growth rate, plant density or the extent of groundcover.

"Plant growth" refers to the growth of any plant part, including stems, leaves and roots. Growth may refer to the rate of growth of any one of these plant parts. The term "yield" refers to the amount of produced biological material and may be used interchangeably with "biomass". For crop plants, "yield" may also mean the amount of harvested material per unit of production or per area (e.g. hectare). Yield may be defined in terms of quantity or quality. Thus an increased yield may refer to a higher graded product (without overall increase in biomass) as well as to an increase in biomass yield. The harvested material may vary from crop to crop, for example, it may be seeds, above-ground biomass, below-ground biomass (e.g. potatoes), roots, fruits, or any other part of the plant which is of economic value.

"Yield" also encompasses yield stability of the plants. "Yield" also encompasses yield potential, which is the maximum obtainable yield under optimal growth conditions. Yield may be dependent on a number of yield components, which may be monitored by certain parameters well known to persons skilled in the art that may vary from crop to crop. For example, breeders are well aware of the specific yield components and the corresponding parameters for the crop they are aiming to improve. For example, key yield parameters for potato include tuber weight, number of tubers, and number of stems per plant.

By "improving soil quality" is meant increasing the amount and/or availability of nutrients required by, or beneficial to plants, for growth. By way of example only, such nutrients include nitrogen, phosphorous, potassium, copper, zinc, boron and molybdenum. Also encompassed by the term "improving soil quality" is reducing or minimizing the amount of an element that may be detrimental to plant growth or development such as, for example iron and manganese. Thus, improving soil quality by use of microbial compositions of the present disclosure thereby assists and promotes the growth of plants in the soil.

The term "crop" as used herein refers to any plant grown to be harvested or used for any economic purpose, including for example human foods, livestock fodder, fuel or pharmaceutical production (e.g. poppies).

The term "identity" in the context of a %-identity of two biological sequences is used herein to describe the percentage of identical (not similar) amino acids or nucleotides of two sequences. A common method to determine the %-identity of two sequences is e.g. the BLAST algorithm (Needleman S.B. and Wunsch C.D. (1970), J. Mol. Biol. 48(3): 443-53) such as implemented in EMBOSS needle (Rice P., Longden I. and Bleasby A. (2000), EMBOSS: The European Molecular Biology Open Software Suite, Trends Genet. 16(6): 276-277) using the documented default configuration.

As used herein, the term "variant" or "variants" refers to both naturally occurring and specifically developed variants or mutants of the microbial strains disclosed and exemplified herein. Also encompassed by the term "variant" as used herein are microbial strains phylogenetically closely related to strains disclosed herein and strains possessing substantial sequence identity with the strains disclosed herein at one or more phylogenetically informative markers such as rRNA genes, elongation and initiation factor genes, RNA polymerase subunit genes, DNA gyrase genes, heat shock protein genes and recA genes. For example, the 16S rRNA genes of a "variant" strain as contemplated herein may share about 90%, 91%, 92%, 93%, 94%, 95%, 96%, 97%, 98% or 99% sequence identity with a strain disclosed herein, and preferably at least 97%. Sequences with greater than 97% identity are typically assigned to the same species (see Patrick D. Schloss and Jo Handelsman, Applied and Environmental Microbiology, Volume 71 Number 3, Pages: 1501-6, March 2005, and referenced cited therein).

Accordingly, one embodiment the microbial composition of the invention comprises one or more variants of the disclosed microbial species or strains. In particular, it is contemplated that the microbial composition comprises one or more variants of the disclosed microbial species or strains having at least about 90%, at least about 91%, at least about 92%, at least about 93%, at least about 94%, at least about 95%, at least about 96%, at least about 97%, at least about 98%, or at least about 99% identity in the 16S rRNA of the corresponding strain.

For instance, in some unclaimed aspects, the microbial composition comprises one or more variants of any one of *Lactobacillus casei, Lactobacillus plantarum, Lactobacillus fermentum, Saccharomyces cerevisiae, Rhodopseudomonas palustris* and *Acetobacter lovaniensis,* having at least about 97% identity in the16S rRNA of the species.

The concentrations of each microbial strain to be added to microbial compositions as disclosed herein will depend on a variety of factors including the identity and number of individual strains employed, the plant species being treated, the nature and condition of the soil to be treated, the exact nature of the microbial composition to be applied, the form in which the composition is applied and the means by which it is applied, and the stage of the plant growing season during which application takes place. For any given case, appropriate concentrations may be determined by one of ordinary skill in the art using only routine experimentation. Concentrations can be given as colony forming units (cfu or CFU) per Millilitre (ml). By way of example, the concentration of each strain present in the inoculant or fertilizer composition may be from about 1 x 10² cfu/ml to about 1 x 10¹⁰ cfu/ml, and may be about 1 x 10³ cfu/ml, about 2.5 x 10³ cfu/ml, about 5 x 10³ cfu/ml, 1 x 10⁴ cfu/ml, about 2.5 x 10⁴ cfu/ml, about 5 x 10⁴ cfu/ml, 1 x 10⁵ cfu/ml, about 2.5 x 10⁵ cfu/ml, about 5 x 10⁵ cfu/ml, 1 x 10⁶ cfu/ml, about 2.5 x 10⁶ cfu/ml, about 5 x 10⁶ cfu/ml, 1 x 10⁷ cfu/ml, about 2.5 x 10⁷ cfu/ml, about 5 x 10⁷ cfu/ml, 1 x 10⁸ cfu/ml, about 2.5 x 10⁸ cfu/ml, about 5 x 10⁸ cfu/ml, 1 x 10⁹ cfu/ml, about 2.5 x 10⁹ cfu/ml, or about 5 x 10⁹ cfu/ml. The final concentration of the strains in the microbial composition may preferably be between about 1 x 10⁴ cfu/ml and 1 x 10⁶ cfu/ml for each species or genus. The final concentration in the inventive microbial composition may therefore range between from about 5 x 10³ to about 2.5 x 10¹⁰ and preferably is 1 x 10⁴ , 2 x 10⁴ , 3 x 10⁴ , 4 x 10⁴ , 5 x 10⁴ cfu/ml or higher.

Microbial compositions of the present disclosure may optionally further comprise one or more additional microbial organisms, for example, additional agronomically beneficial microorganisms. Such agronomically beneficial microorganisms may act in synergy or concert with, or otherwise cooperate with the organisms of the present disclosure in the composition. Examples of agronomically beneficial microorganisms include *Bacillus* sp., *Pseudomonas* sp., *Rhizobium* sp., *Azospirillum* sp., *Azotobacter* sp., phototrophic and cellulose degrading bacteria, *Clostridium* sp., *Trichoderma* sp. and the like.

Those skilled in the art will appreciate that any plant may benefit from the application of microbial compositions of the present disclosure to soil, seeds and/or vegetation. Particular embodiments are employed to aid the growth, development, yield or productivity of crops and pastures or other plants of economic value, including ornamentals and plants grown for oils or biofuel. The crop plant may be, for example, a food crop (for humans or other animals) such as any fruit, vegetable, nut, seed or grain producing plant.

Exemplary crop plants include, but are not limited to, tubers and other below-ground vegetables (such as potatoes, beetroots, radishes, carrots, onions, etc.), ground-growing or vine vegetables (such as pumpkin and other members of the squash family, beans, peas, asparagus, etc.), leaf vegetables (such as lettuces, chard, spinach, alfalfa, etc.), other vegetables (such as tomatoes, brassica including broccoli, avocadoes, etc.), fruits (such as berries, olives, stone fruits including nectarines and peaches, tropical fruits including mangoes and bananas, apples, pears, mandarins, oranges, mandarins, kiwi fruit, coconut, etc.), cereals (such as rice, maize, wheat, barley, millet, oats, rye etc.), nuts (such as macadamia nuts, peanuts, brazil nuts, hazel nuts, walnuts, almonds, etc.), and other economically valuable crops and plants (such as sugar cane, soybeans, sunflower, canola, sorghum, pastures, turf grass, etc).

Microbial compositions of the present disclosure may be applied directly to plants, plant parts (such as foliage) or seeds, or alternatively may be applied to soil in which the plants are growing or to be grown or in which seeds have been or are to be sown. Application may be by any suitable means and may be on any suitable scale. For example, application may comprise pouring, spreading or spraying, including broad scale or bulk spreading or spraying, soaking of seeds before planting, and/or drenching of seeds after planting or seedlings. Multiple means of application may be used in combination (for example soaking of seeds prior to planting followed by drenching of planted seeds and/or application to seedlings or mature plants). Seeds, seedlings or mature plants may be treated as many times as appropriate. The number of applications required can readily be determined by those skilled in the art depending on, for example, the plant in question, the stage of development of the plant at which treatment is initiated, the state of health of the plant, the growth, environmental and/or climatic conditions in which the plant is grown and the purpose for which the plant is grown. For example, in the case of flowering crops such as tomatoes, it may be desirable to apply the microbial composition once or more than once during the flowering period.

The microbial compositions as disclosed herein may be prepared in any suitable form depending on the means by which the composition is to be applied to the soil or to plant seeds or vegetation. Suitable forms can include, for example, slurries, liquids, and solid forms. Solid forms include powders, granules, larger particulate forms and pellets (for example dyed or undyed gelatine spheres or capsules). Liquids may include aqueous solutions and aqueous suspensions, and emulsifiable concentrates.

In order to achieve effective dispersion, adhesion and/or conservation or stability within the environment of the microbial compositions disclosed herein, it may be advantageous to formulate the compositions with suitable carrier components that aid dispersion, adhesion and/or conservation/stability. Suitable carriers will be known to those skilled in the art and include, for example, ceramics or ceramic powder, chitosan, vermiculite, compost, talc, chalk, carbon particles or powder, charcoal, rice hulls, gels and the like.

Additional components may be incorporated into compositions of the present disclosure, such as humic substances, trace elements, organic material, penetrants, macronutrients, micronutrients and other soil and/or plant additives. Humus or humic substances that may be incorporated may include, but are not limited to, humic acid derived from, for example oxidised lignite or leonardite, fulvic acid and humates such as potassium humate. Organic material added may include, but is not limited to, biosolids, animal manure, compost or composted organic byproducts, activated sludge or processed animal or vegetable byproducts (including blood meal, feather meal, cottonseed meal, ocean kelp meal, seaweed extract, fish emulsions and fish meal). Penetrants include, but are not limited to, non-ionic wetting agents, detergent based surfactants, silicones, and/or organo-silicones. Suitable penetrants will be known to those skilled in the art, non-limiting examples including polymeric polyoxyalkylenes, allinol, nonoxynol, octoxynol, oxycastrol, TRITON, TWEEN, Sylgard 309, Silwet L-77, and Herbex (silicone / surfactant blend).

For the methods of the present invention, the microbial composition may be formulated in any appropriate form for the application to plants or as soil treatment and can be applied at any appropriate time, or in any appropriate time intervals depending on the use case. Typically, for the treatment of plants growing on land plots about 0,1 to about 50 L, preferably about 0,5 to about 25 L and in particular about 1-5 to about 10-20 L per ha of a microbial composition containing a CFU/ml as described above may be used. For soil treatments about 0,3-0,5 to about 300 L, preferably about 1 to about 150 L and in particular about 2-10 to about 40-60 L per ha of a microbial composition containing a CFU/ml as described above may be used. If use as a growth enhancer, the microbial compositions may typically be applied a plurality of times before and/or during the growth period of the plants, depending on the soil type and its agricultural history (previous agricultural use, previous fertilizing regime, etc.). The application may occur once before and/or after planting and/or once or several times weekly/monthly. For pathogen control, the application of the microbial compositions may be applied prophylactically once or more times, or may be applied only when an infection with the pathogen is detected. For acute pathogen treatment, the microbial compositions may be used once, or a plurality of times until repression of the pathogen or until time for harvest of the plants.

Plant pathogens and the associated plant diseases that may effectively be treated by the microbial compositions of the present invention include, but do not form part of the invention (Table 2):

**Table 2**

| **Crop** | **Pathogen** | **Disease common name** |
|---|---|---|
| Banana and Plaintain | banana bunchy top virus (BBTV) | Banana bunchy top |
| | *Mycosphaerella fijiensis* | black sigatoka |
| | *Fusarium oxysporum* f.sp. *cubense* | Panama disease |
| Barley | *Fusarium graminearum* | Fusarium head blight |
| | *Blumeria graminis* f. sp. *hordei* | powdery mildew |
| | *Puccinia graminis* f. sp. *hordei* | barley stem rust |
| Cassava | African cassava mosaic virus (ACMVD) | African cassava mosaic disease |
| | *Xanthomonas axonopodis* pv. *manihotis* | bacterial blight |
| | Cassava brown streak virus (CBSV) | cassava brown streak disease |
| Cotton | *Xanthomonas citri* pv. *malvacearum* | bacterial blight |
| | *Fusarium oxysporum* f. sp. *vasinfectum* | Fusarium wilt |
| | *Verticillium dahliae* | Verticillium wilt |
| Maize/corn | *Aspergillus flavus* | Aspergillus ear rot |
| | *Fusarium graminearum* | Giberella stalk and ear rot |
| | *Cercospora zeae-maydis* | grey leaf spot |
| Palm fruit | *Ganoderma orbiforme*/*Ganoderma boninense* | Basal stem rot |
| | *Phytophthora palmivora* | bud rot |
| Peanut | Groundnut rosette virus (GNV) | Groundnut rosette disease |
| | Groundnut rosette assistor virus (GRAV) | |
| Potato | *Ralstonia solanacearum* | Potato brown rot |
| | *Phytophthora infestans* | late blight |
| Rapeseed and mustard | *Leptosphaeria maculans* | Phoma stem canker |
| | *Sclerotinia sclerotiorum* | Sclerotinia stem rot |
| Rice | *Magnaporthe oryzae* | rice blast |
| | *Xanthomonas oryzae* pv*. oryzae* | rice bacterial blight |
| | *Rhizoctonia solani* | sheath blight |
| Sorghum and millet | *Colletotrichum sublineolum* | Anthracnose |
| | *Exserohilum turcicum* | Turcicum leaf blight |
| Soybean | *Heterodera glycines* | soybean cyst nematode disease |
| | *Phakopsora pachyrhizi* | Asian soybean rust |
| Sugar beet | *Cercospora beticola* | Cercospora leaf spot |
| | Beet necrotic yellow vein virus (BNYVV) | rhizomania |
| Sugarcane | *Leifsonia xyli* subsp. *xyli* | Ratoon stunting |
| | *Colletotrichum falcatum* | red rot |
| Sweet potato | Sweet potato feathery mottle virus (SPFMV) | sweet potato virus disease (SPVD) |
| | Sweet potato chlorotic stunt virus (SPCSV) | |
| Tomato | *Phytophthora infestans* | late blight |
| | Tomato yellow leaf curl virus (TYLCV) | tomato yellow leaf curl |
| Wheat | *Fusarium graminearum* | *Fusarium* head blight |
| | *Puccinia graminis* | wheat stem rust |
| | *Puccinia striiformis* | wheat yellow rust |
| Yam | *Colletotrichum gloeosporioides* | anthracnose |
| | Yam mosaic virus (YMV) | yam mosaic disease |

In particular, the microbial compositions disclosed herein are useful to protect a plant from infection / treat a plant afflicted with *F. poae.* In further unclaimed aspects the disclosure relates to the microbial compositions useful to protect a plant from infection / treat a plant afflicted with (a) a fungal pathogens selected from *Pestalopsis spp., Neopestalotiopsis spp., Fusarium spp.* such as *F. avenaceum, F. culmorum, F. graminearum, , F. oxysporum* and special forms thereof including *F. oxysporum f. sp. zingiberi, ~ f. sp. niveum,* and ~ *f. sp, cubense; Collectotrichum coccodes: Phytophthora spp.* such as *Phytophthora infestans, Phytophthora erythroseptica, Phytophthora cinnamomi; Rhizoctonia spp.* such as *Rhizoctonia solani; Corynespora cassiicola; Puccinia spp.* such as *Puctinia arachidus, Puccinia striiformis, Puccinia graminis f. sp. tritici; Botrytis cinerea; Rhizoctonia; Pythium ultimum, Pythium myriotylum; Psuedocercospora macadamiae; Rosellinia necartrix; Verticillium spp.* such as *Verticillium deliliae; Phialemonium dimorphosporum; Thielaviopsis spp.; Magnaporthe grisea, Sclerotinia sclerotiorum, Ustilago spp., Phakospora pachyrhizi,* and *Armillaria spp.* or (b) a bacterial pathogen selected from *Phytoplasma spp., Spiroplasma spp., Streptomyces scabies; Xanthomonas spp.* such as *Xanthomonas campestris pv. Vesicatoria; Pseudomonas spp.* such as *P*. *savastanoi, P. syringae, P. aeruginosa,* and *P*. *fluorescens; E. coli; Listeriamonocytogenes; Staphylococcus spp.* such as *S. aureus; Streptococcus spp.* such as *S*. *uberis; Agrobacterium tumefaciens; Burkhoideria cepacia; Erwinia carotovora; Erwinia chrysanthemi; Pantoea agglomerons; Ralstonia solanacearum; Pantoea stewartii; Aeromonas hydrophila; Vibrio spp.* such as *V. anguillarum, V. harveyi, V. cholera,* and *V. parahaemoliticus; Actinobacillus pleuropneumoniae; Chromobacter violaceum; Coxiella burnetti; Francisella tularensis; Haemophilus influenza; Pasteurella multocida; Shigella flexneri; Salmonella typhi; Salmonella typhimurium; Yersinia pestis;* and *Yersinia pseudotuberculosis.*

The following examples demonstrate the efficacy of the microbial compositions of the present invention in the treatment and prevention of various plant infections caused by a number of plant pathogens. In addition, the examples show that the field application of the microbial compositions of the present invention exhibit a growth promoting effect on certain crop plants.

The microbial compositions used in the Examples are exemplary compositions and shall not be construed to be limiting on the present invention.

### Examples

In the following examples, microbial compositions (e.g. Product 1, Product 2 and Product 3) were used which contain *Lactobacillus casei, Lactobacillus plantarum, Lactobacillus fermentum, Saccharomyces cerevisiae, Rhodopseudomonas palustris,* and *Acetobacter lovaniensis.*

Dependent on the mode of application (e.g. in agar use, foliar, soil treatment), the microbial compositions were supplemented with additional species as outlined in the summary of the invention.

While positive results have been obtained with compositions with subgroups of these species the present examples provide a best mode of the present invention.

### Example 1. Late blight field trial (reference example)

The impact of the microbial compositions on the control of late blight (*Phytophthora infestans*) in potatoes was investigated in a field trial. A separate, fully replicated micro-plot trial was established as part of a larger commercial trial (400+ plots). Each plot was 6 metres by 4 rows of Maris Piper potatoes, grown to full commercial yield and with standard conventional inputs apart from fungicides.

For this trial, there were seven treatments with four replicates for each treatment. Treatments were only applied on 7 days intervals, regardless of disease pressure. The pathogen was introduced into the plots 30 days after emergence. The percentage of leaf area damaged by lesions was assessed weekly.

Treatments: 1. Untreated control; 2. MK5 3L/ha; 3. Product 1 20L/ha + 3kg Ceramic powder; 4. Product 1 10L/ha + 3kg Ceramic powder; 5. *B. amyloliquefaciens* + wetter; 6. Phosphite and chemical fungicide; 7. Chemical fungicide. MK5 is a fermentation product of garlic and chili used in organic agriculture.

The results are given in the following table (and Figures 1A and 1B); the values are given as % leaf area lesions:

**Table 3**

| *Treatment* | *09*/*07*/*10* | *16*/*07*/*10* | *02*/*08*/*10* | *06*/*08*/*10* | *11*/*08*/*10* | *16*/*08*/*10* | *21*/*08*/*10* | *26*/*08*/*10* |
|---|---|---|---|---|---|---|---|---|
| 1 | 0 | 0 | 0.225 | 0.325 | 0.65 | 1.275 | 22 | 51.25 |
| 2 | 0 | 0 | 0.025 | 0.125 | 0.3 | 0.425 | 1.625 | 10.25 |
| 3 | 0 | 0 | 0.075 | 0.125 | 0.3 | 0.375 | 2.75 | 12.25 |
| 4 | 0 | 0 | 0 | 0.025 | 0.15 | 0.375 | 3.75 | 19.25 |
| 5 | 0 | 0 | 0.025 | 0.1 | 0.175 | 0.45 | 6 | 19.75 |
| 6 | 0 | 0 | 0.025 | 0.05 | 0.3 | 0.6 | 0.9 | 2.25 |
| 7 | 0 | 0 | 0.025 | 0.175 | 0.425 | 0.65 | 1.15 | 3 |

Treatment 1 marks the untreated control. The plots exhibited prominent late blight infection with over 50% of leaf area lesions. Treatment 6 and 7 mark the classical chemical approach of disease control (with all its drawbacks as discussed in the introductory portion). Treatment 5 marks a treatment with one single strain that was found beneficial in the microbial compositions of the present invention. Treatments 2-4 are treatments with different inventive microbial compositions. All of the compositions perform better than the single strain treatment 5. For the composition of Product 1, a dose dependent effect was observed (compare treatments 3 and 4). Product 1 and MK5 were able to reduce leaf lesion area by the factor of about 5 as compared to the untreated control and demonstrate that they are able to strongly inhibit pathogen growth (see Fig. 1B).

### Example 2. Macadamian diseases (reference examples)

Preliminary laboratory testing of the inventive microbial compositions against selected macadamian diseases was performed. The microbial compositions inhibited *Neopestalotiopsis* infections at all concentrations investigated.

### Example 3. Effect against Colletotrichum coccodes and Rhizoctonia solani (reference examples)

The efficacy of a microbial composition of the invention (Product 1) against the funghi *Colletotrichum coccodes* and *Rhizoctonia solani* was investigated. Outline methodology: The fungi were introduced to agar plates containing three different concentrations of Product 1; dilutions were made with water. (- 1 = 0.1%; -3 = 0.001%; -5 = 0.00001%). There were 8 - 10 replicates for each species and treatment combination.

There was a single point of inoculum on day '0' from which radial growth was measured daily for 8 - 10 days.

**Table 4**

| ***Colletotrichum coccodes*** with Product 1 | | | | | |
|---|---|---|---|---|---|
| | **Day** | **2** | **3** | **6** | **7** |
| | **control** | 4.2 | 7.75 | 18.7 | 23.1 |
| | **-1** | 2.25 | 2.5 | 2.5 | 2.5 |
| | **-3** | 4.25 | 6.7 | 6.7 | 6.7 |
| | **-5** | 4.25 | 7.8 | 19.55 | 24.3 |

| ***Rhizoctonia solani*** with Product 1 | | | | | |
|---|---|---|---|---|---|
| | **Day** | **2** | **3** | **6** | **7** |
| | **control** | 9.75 | 20.05 | 39.45 | n.d. |
| | **-1** | 3.1875 | 4.75 | 4.75 | 4.75 |
| | **-3** | 7.85 | 10.45 | 10.45 | 10.45 |
| | **-5** | 9.2 | 17 | 25.05 | 27.3 |

Result summary (see Figure 2): Both fungi were sensitive to Product 1 and at the higher concentrations, there was little or no growth. The Rhizoctonia was the more vigorous in growth but also the most susceptible.

The results demonstrate that Product 1 is an effective measure against the two serious plant pathogens *Colletotrichum coccodes* and *Rhizoctonia solani;* the former causing anthracnose on tomato and black dot disease of potato, the latter causing many plant diseases such as 'Damping Off' and 'Crown Rot'.

### Example 4. Effect against Fusarium graminearum, F. oxysporum (reference examples) and F. poae (inventive)

The efficacy of a microbial composition of the invention (Product 1) against the important foliar plant pathogens of *Fusarium graminearum, F. oxysporum, and F. poae* was investigated. All were inhibited in a concentration dependent manner. Outline methodology: The fungi were introduced to agar plates containing three different concentrations of Product 1 (- 1 = 0.1%; -3 = 0.001%; -5 = 0.00001%). There were 8 - 10 replicates for each species and treatment combinations. There was a single point of inoculum on day '0' from which radial growth was measured daily for 8 - 10 days.

### Results:

**Table 5**

| *F. oxysporum* with Product 1 | | | | | | | |
|---|---|---|---|---|---|---|---|
| | **Day** | **1** | **2** | **3** | **6** | **8** | |
| | **control** | 2 | 4.7 | 8.5 | 26.9 | 38.35 | |
| | **-1** | 1.5 | 2.9 | 5.3 | 9.1 | 12.2 | |
| | **-3** | 1.75 | 4.2 | 6.05 | 13.4 | 17.7 | |
| | **-5** | 1.8 | 4.85 | 8.25 | 19.3 | 26.95 | |

| *F. graminearum* with Product 1 | | | | | | | |
|---|---|---|---|---|---|---|---|
| | **Day** | **3** | **4** | **5** | **6** | **7** | **10** |
| | **control** | 8.125 | 13.375 | 18.375 | 23.375 | 28.5 | 38.5 |
| | **-1** | 2.75 | 6.875 | 7.5 | 8.875 | 8.875 | 9.125 |
| | **-3** | 5.25 | 8.75 | 10.625 | 11 | 13.625 | 14.5 |
| | **-5** | 6.5 | 10.25 | 11.25 | 13.125 | 13.875 | 17.5 |

| *F. poae* with Product 1 | | | | | | | |
|---|---|---|---|---|---|---|---|
| | **Day** | **3** | **4** | **5** | **6** | **7** | **10** |
| | **control** | 12.125 | 17.375 | 23.25 | 29.875 | 33.5 | n.d. |
| | **-1** | 8 | 9.25 | 9.5 | 10.625 | 10.625 | 11.375 |
| | **-3** | 11.625 | 17.25 | 26.875 | 33 | 37.625 | n.d. |
| | **-5** | 10.875 | 20 | 28.125 | 31.875 | 39.125 | n.d. |

| | | | | | | | |
|---|---|---|---|---|---|---|---|
| n.d.: not determined | | | | | | | |

All Fusarium species tested were inhibited by Product 1 to a greater or lesser degree, depending on the concentration (see Figure 3). The most susceptible was *F. graminearum* and the least was *F. poae.* At the highest concentration investigated (0.1%), none of the species tested exceeded 12 mm of radial growth.

### Example 5. Effect against Fusarium avenaceum, F. culmorum, and Pythium ultimum

The efficacy of a microbial composition of the invention (Product 1) against the important plant pathogens of *Fusarium avenaceum, F. culmorum,* and *Pythium ultimum* was investigated. All were inhibited in a dose dependent manner. Outline methodology: The fungi were introduced to agar plates containing three different concentrations of Product 1 (- 1 = 0.1%; -3 = 0.001%; -5 = 0.00001%). There were 8 - 10 replicates for each species and treatment combinations. There was a single point of inoculum on day '0' from which radial growth was measured daily for 8 - 10 days.

### Results:

**Table 6**

| ***F. avenaceum* with Product 1** | | | | | | | | |
|---|---|---|---|---|---|---|---|---|
| | **Day** | **1** | **4** | **5** | **6** | **7** | **8** | **9** |
| | control | 1.9 | 12.35 | 17.25 | 19.9 | 22.45 | 23.65 | 26.3 |
| | -1 | 1 | 5.45 | 5.45 | 5.45 | 5.45 | 5.45 | 5.45 |
| | -3 | 1.35 | 9.8 | 13 | 14.95 | 16.15 | 17.65 | 19.85 |
| | -5 | 1.9 | 11.95 | 16.9 | 19.95 | 22.7 | 23.6 | 25.8 |

| ***F. culmorum* with Product 1** | | | | | | | | |
|---|---|---|---|---|---|---|---|---|
| | **Day** | **1** | **4** | **5** | **6** | | | |
| | control | 3.05 | 33.2 | 39.9 | | | | |
| | -1 | 2.55 | 6.5 | 6.5 | 6.5 | | | |
| | -3 | 2.95 | 19 | 25.55 | 30.95 | | | |
| | -5 | 3 | 32.95 | 39.9 | n.d. | | | |

| ***Pythium ultimum* with Product 1** | | | | | | | | |
|---|---|---|---|---|---|---|---|---|
| | **Day** | **1** | **2** | **3** | **6** | **8** | | |
| | control | 12.3 | 31.75 | 39.95 | n.d. | n.d. | | |
| | -1 | 9.5 | 12.15 | 15.4 | 30.8 | 39.7 | | |
| | -3 | 13.15 | 32.1 | 40 | n.d. | n.d. | | |
| | -5 | 12.3 | 30.25 | 40.05 | n.d. | n.d. | | |

| | | | | | | | | |
|---|---|---|---|---|---|---|---|---|
| n.d.: not determined | | | | | | | | |

The results are also shown in Figure 4. All three fungi were inhibited by Product 1 at the greatest concentration investigated (0.1%). The most susceptible was *F. avenaceum* and the least was *Pythium ultimum.* At a concentration of 0.1%, none of the species tested exceeded 12 mm of radial growth.

### Example 6. Antagonistic effect of microbial lead strains (Supplement 1) against Fusarium poae

Inhibition experiments were carried out to establish potential antagonistic activity of the microbial components of a microbial composition of the invention (Product 1) to selected plant pathogens. Outline methodology: Inhibition assays were carried on Potato Dextrose Agar (PDA) to determine the effects of the microbial components of Product 1 (containing *Lactobacillus casei, L. fermentum, L. plantarum, Saccharomyces cerevisiae* and *Rhodopseudomonas palustris*) on pathogen targets. A volume (100µl) of each microbial component (minimum of 10⁵/ml) of Product 1 was spread separately onto half strength PDA, which favours fungal growth. A fungal plug taken from an actively growing culture of the target pathogen was placed in the centre of the agar plate, and the radial growth of the fungal plug was monitored at regular intervals.

### Results:

**Table 7**

| *Lactobacillus casei* | | | | | | | | | | |
|---|---|---|---|---|---|---|---|---|---|---|
| **Days** | **3** | **5** | **7** | **10** | **12** | **14** | **17** | **19** | **25** | **30** |
| control | 7.125 | 21.3125 | 30.625 | 41 | n.d. | n.d. | n.d. | n.d. | n.d. | n.d. |
| -1 | 6.0625 | 8.0625 | 10.3125 | 13.1875 | 14.125 | 15.875 | 17.5625 | 18.3125 | 19.5 | 21.812 |
| -2 | 5.25 | 7.375 | 9.9375 | 12.8125 | 13.9375 | 16 | 16.875 | 17.1875 | 19.625 | 21.5 |
| -3 | 7.125 | 8.4375 | 10.6875 | 14.875 | 16.375 | 18.375 | 19.375 | 19.25 | 21.5 | 22.875 |
| -4 | 7.25 | 9.0625 | 10.25 | 13.375 | 14.625 | 16 | 17.8125 | 17.625 | 21.187 | 22.812 |
| -5 | 7.1875 | 8 | 9.25 | 12.5 | 14.5625 | 16.875 | 17.125 | 17.5 | 20.812 | 24.519 |

| *L. plantarum* | | | | | | | | | | |
|---|---|---|---|---|---|---|---|---|---|---|
| **Days** | **3** | **5** | **7** | **10** | **12** | **14** | **17** | **19** | **25** | **30** |
| control | 7.125 | 21.3125 | 30.625 | 41 | n.d. | n.d. | n.d. | n.d. | n.d. | n.d. |
| -1 | 7.875 | 7.75 | 10.3125 | 13.625 | 16.3125 | 18.1875 | 21.25 | 23.6875 | 25.0625 | 25 |
| -2 | 7.5 | 7.875 | 10.0625 | 12.4375 | 13.6875 | 15 | 16.625 | 17.125 | 19.9375 | 20.25 |
| -3 | 8.3125 | 8.5 | 10.9375 | 15.3125 | 17.0625 | 19.0625 | 19.5 | 19.625 | 21.875 | 22.1875 |
| -4 | 7.5625 | 8.4375 | 10.5625 | 13.4375 | 14.75 | 16.625 | 16.75 | 17.375 | 19.375 | 20.1875 |
| -5 | 7.4375 | 8.5625 | 10.6875 | 13.25 | 15.5 | 17.3125 | 18.375 | 18.9375 | 21.1875 | 22.125 |

| *L. fermentum* | | | | | | | | | | |
|---|---|---|---|---|---|---|---|---|---|---|
| **Days** | **3** | **5** | **7** | **10** | **12** | **14** | **17** | **19** | **25** | **30** |
| control | 7.125 | 21.3125 | 30.625 | 41 | n.d. | n.d. | n.d. | n.d. | n.d. | n.d. |
| -1 | 7.8125 | 8.75 | 11.125 | 14.25 | 15.8125 | 17.9375 | 18.25 | 18.25 | 21.9375 | 22.8125 |
| -2 | n.d. | n.d. | n.d. | n.d. | n.d. | n.d. | n.d. | n.d. | n.d. | n.d. |
| -3 | 7.8125 | 8.5625 | 10.625 | 14.5625 | 16.3125 | 18.0625 | 19 | 18.125 | 21.75 | 23 |
| -4 | 7.25 | 8 | 10 | 12.75 | 14.25 | 15.75 | 16.75 | 16.5 | 19.25 | 21 |
| -5 | 7.5625 | 8.25 | 9.8125 | 13.6875 | 15 | 16.875 | 18.9375 | 18.8125 | 20.5625 | 23.25 |

| *Saccharomyces cerevisiae* | | | | | | | | | | |
|---|---|---|---|---|---|---|---|---|---|---|
| **Days** | **3** | **5** | **7** | **10** | **12** | **14** | **17** | **19** | **25** | **30** |
| control | 7.125 | 21.3125 | 30.625 | 34.5 | 38.3125 | 40.5625 | 41 | n.d. | n.d. | n.d. |
| -1 | 7.5625 | 9.5 | 10.5 | 11.4375 | 12.5625 | 16.4375 | 18.875 | 21.625 | 25 | 28.0625 |
| -2 | 6.75 | 7.3125 | 7.5 | 7.1875 | 9.0625 | 11.4375 | 13.6875 | 16.5 | 20 | 22.9375 |
| -3 | n.d. | n.d. | n.d. | n.d. | n.d. | n.d. | n.d. | n.d. | n.d. | n.d. |
| -4 | 7 | 8.625 | 13.5 | 13.625 | 15 | 18.125 | 20.75 | 23.5 | 26.75 | 30 |
| -5 | 4.9375 | 7.9375 | 10.75 | 11.8125 | 14.3125 | 17.1875 | 19.9375 | 22.8125 | 25.9375 | 29.5 |

| *Rhodopseudomonas palustris* | | | | | | | | | | |
|---|---|---|---|---|---|---|---|---|---|---|
| **Days** | **3** | **5** | **7** | **10** | **12** | **14** | **17** | **19** | **25** | **30** |
| control | 7.125 | 21.3125 | 30.625 | 34.25 | 39.8125 | 41 | n.d. | n.d. | n.d. | n.d. |
| -1 | 5.75 | 8 | 10 | 9.75 | 11.25 | 12.875 | 14.75 | 16.625 | 20.4375 | 24.5625 |
| -2 | 5.5 | 8.5 | 10.5 | 10.9375 | 12.3125 | 13.625 | 15.8125 | 17.875 | 21.875 | 25.875 |
| -3 | 7 | 8.125 | 10.6875 | 10.5 | 12.3125 | 13.875 | 15.8125 | 18 | 21.6875 | 25.8125 |
| -4 | 6.875 | 11.1875 | 13.875 | 14 | 15.5625 | 17.1875 | 19 | 21.375 | 25.3125 | 29.125 |
| -5 | 5.375 | 12.75 | 14.5 | 18.9375 | 20.6875 | 22.4375 | 24.0625 | 26.375 | 29.9375 | 33.3125 |

| | | | | | | | | | | |
|---|---|---|---|---|---|---|---|---|---|---|
| *n.d.: not determined, : control = PDA without lead strain, - 1 = dilution by 10; -2= dilution by 100, -3 = dilution by 1000; -4= dilution by 10000 -5 = dilution by 100000.* | | | | | | | | | | |

The results are also shown in Figure 5. The effect of co-incubation of the microbial components of Product 1 and *Fusarium poae* was recorded as the effect of rate and extent of radial growth of the pathogen. Co-incubation with each of the five microbial components resulted in a significant reduction in the pathogen's rate of growth at all concentrations. Growth on the control plates reach the maximum level after about 10 days, whereas for the majority of the treatments, pathogen growth was only half this level after 30 days.

### Example 7. Antagonistic effect of secondary microbial strains (Supplements 2a, 2b, 3) against

### Fusarium culmorum, F. sulphureum and F. coeruleum (reference example)

A further laboratory study assessed the antagonistic activity of some of the secondary bacterial species in the Product 1 consortium against *Fusarium culmorum, F. sulphureum* and *F*. *coeruleum.*

All investigated secondary species (*Bacillus subtilis, Bacillus amyloliquefaciens, Bacillus licheniformis, Bacillus megaterium, Acetobacter indonesiensis, Acetobacter estunensis, Pediococcus acidilactici, Pseudomonas putida, Azotobacter vinelandii)* show an additional positive effect on the suppression of the different *Fusarium* spp.

### Example 8. Improving ground cover of Kale crops by pre transplant treatment (reference example)

In a field study the impact of an inventive microbial composition (Product 2) on Kale plugs was investigated. It has been shown that the microbial composition had benefits for Kale crop when used pre transplant.

Methodology: Kale plugs were dipped in different rates of Product 2 and transplanted into the field. Fully replicated trial plots of 18 plus plants per plot were assessed for ground coverage (three plots per concentration of Product 2 were used). No statistically significant differences were identified in the data due to variation within the single plots. However, the dipped plants at 50% and 100% of recommended rate were visibly better looking, which is reflected in the early ground cover measurements taken 2 weeks after transplant (see Figure 6). There were indications of mild phytotoxic effect with the two higher rates (200% and 300%).

Clearly, the benefits of the application of the microbial composition on the implantation of Kale crops lies in the early stages, as would be expected, and where there is an increased risk of unexpected stresses. That is, the inventive microbial compositions provide the advantage of yielding more robust transplants.

### Example 9. Improving yield of pea (Pisum sativum) (reference example)

In a field trial (strip trial) in the middle of commercial crops, the impact on the microbial compositions for use as a fertilizer / soil conditioner was investigated.

### Methodology:

Variety: Oasis
Site: Strip trial in middle of commercial crop
Soil type: Silt
Product 2 was applied at planting at a rate of 50L/ha in 200L water

Triplicates of field plots were investigated.

### Results:

**Table 8**

| | **t/ha** | | | | |
|---|---|---|---|---|---|
| | **whole crop above ground** | **pod number/ha** | **Whole pod weight** | **Ungraded Fresh pea** | **Pea weight/1 pod** |
| | t/ha | th/ha | t/ha | t/ha | g |
| 50L Product 2 at planting | 38.4 | 4375.0 | 20.9 | 10.1 | 2.3 |
| 50L Product 2 at planting | 39.4 | 4600.0 | 19.5 | 11.1 | 2.4 |
| 50L Product 2 at planting | 43.2 | 4633.3 | 21.1 | 10.4 | 2.3 |
| *Mean* | *40.3* | *4536.1* | *20.5* | *10.6* | *2.3* |
| St err | 1.5 | 81.2 | 0.5 | 0.3 | 0.0 |
| Untreated | 31.8 | 3700.0 | 15.1 | 8.1 | 2.2 |
| Untreated | 33.3 | 4300.0 | 17.2 | 9.3 | 2.2 |
| Untreated | 39.8 | 4000.0 | 18.9 | 9.3 | 2.3 |
| *Mean* | *34.9* | *4000.0* | *17.0* | *8.9* | *2.2* |
| St err | 2.5 | 173.4 | 1.1 | 0.4 | 0.0 |

The results are also shown in Figure 7. The application of a microbial composition of the invention (Product 2) lead to a significantly greater above ground crop than the standard farm practice. The treated area produced significantly greater final fresh weight in Peas and pod numbers per ha than the untreated control area. In addition, the Pea weight per pod was slightly greater in the Product 2 treated area.

The results show that the application of the microbial composition to soil has largely beneficial effects on the growth and yield of *P*. *sativum.*

### Example 10. Microbial compositions as fertilizer substitutes and or fertilizer supplements (reference example)

In a field trial with potatoes (*Solanum tuberosum*) the efficacy of the microbial compositions as activators of the nutrient potential of the soil was investigated. The aim of the trial was to reduce or obviate chemical fertiliser applications, and increase yield and quality of a potato crop using a microbial composition of the invention (Product 2) at planting and using another microbial composition for spraying the plants at a later time point (Product 3). Both products contain *Lactobacillus casei, L. fermentum, L. plantarum, Saccharomyces cerevisiae* and *Rhodopseudomonas palustris* and were conditioned for application to soil (Product 2) or in conjunction with fine ground pure Calcium Carbonate (>99% purity) sprayed onto the leaf surface (Product 3).

**Methodology:** The replicated microplot study sited within a large field of commercial crop (segregated area within a commercial potato crop). For each treatment, three replicates were made. The soil type was silt. The plot size was 5m x 2 rows. The variety of the potato was Nectar.

### Treatments:

1. Product 2 program. Product 2 50L/ha at planting. Product 3 3L/ha + Calcium carbonate 3kg/ha (3 apps) - (reduced fertiliser area)
2. Product 2 program. Product 2 50L/ha at planting. Product 3 3L/ha + Calcium carbonate 3kg/ha (3 apps) plus 100N - (reduced fertiliser area)
3. Product 2 program. Product 2 50L/ha at planting. Product 3 3L/ha + Calcium carbonate 3kg/ha (3 apps) plus Standard Farm Practise
4. Reduced fertiliser - Control (this included as nutrition: 100N 50P 275K)
5. Standard Farm Practise (SFP; this included as nutrition: 200N 150P 275K)

Treatment were applied at the following time points:
T0 - at planting (02.05.20)
T1 - (pre tuber initiation) 03.06.20
T2 - 19.06.20
T3 - 02.07.20 (ground cover 60%, meeting down the rows)

### Results:

The results are also shown in Fig. 8 (treatments 1 to 5 are given in the graphs from left to right). No statistically significant difference was seen in the tuber number and yield data, except the smaller size grading of <45mm, where treatment 2 (Product 2 with 100N) produced significantly more in number and weight than the reduced fertiliser alone treatment. It also produced more in yield at <45mm than the standard farm practise. Therefore, if left a little longer in the field they likely would have moved up a grade and made a significant difference in the final yield (unfortunately, the decision to de-haulm the crop was dependent on the commercial crop in which the test plots were sited). SFP (treatment 5) had, together with treatments 2 and 3 the greatest overall yield. Treatments 2 & 3 (Product 2 treatments) and 5 had the greater total tuber number.

Skin finish was assessed on the tubers but they were fairly clean, with only minor common scab issues.

SAP analysis during crop growth resulted in statistically significant greater levels of NO₃ and NH₄ found in the Product 2 with SFP, than the other tested treatments. It also had significantly less Al than the reduced treatment. There was significantly less Mg and Na in the Product 2 treatment with SFP compared to the other Product 2 treatments and the reduced treatment, indicating that the microbial compositions improve the sustainability of SFP by reducing the accumulation or release of cationic soil minerals.

Overall, the results show that the microbial composition significantly improves the availability of nutrients when used alone and allows using reduced amounts of fertilizer in order to provide commercially competitive yields as compared with SFP.

## Claims

1. Use of a microbial composition comprising *Lactobacillus casei, Lactobacillus plantarum, Lactobacillus fermentum, Saccharomyces cerevisiae, Rhodopseudomonas palustris* and *Acetobacter lovaniensis* for treating or preventing an infection of a plant with *Fusarium poae.*

2. A method for treating or preventing an infection of a plant with a plant pathogen, the method comprising administering to the plant, the seed of the plant, the soil the plant is rooted in, or otherwise exposing the plant, or the seed of the plant to an effective amount of a microbial composition comprising *Lactobacillus casei, Lactobacillus plantarum, Lactobacillus fermentum, Saccharomyces cerevisiae, Rhodopseudomonas palustris* and *Acetobacter lovaniensis,* thereby treating the plant or preventing infection of the plant, wherein the infection is an infection with *F*. *poae.*

3. The use of claim 1, or the method of claim 2, wherein (i) the *Lactobacillus casei* is *Lactobacillus casei* DSMZ 20011, and/or (ii) the *Lactobacillus plantarum* is *Lactobacillus plantarum* DSMZ 21074, and/or (iii) the *Lactobacillus fermentum* is *Lactobacillus fermentum* DSMZ 20052, and/or (iv) the *Saccharomyces cerevisiae* is *Saccharomyces cerevisiae* DSMZ 70449, and/or (v) the *Rhodopseudomonas palustris* is *Rhodopseudomonas palustris* ATCC 17001, and/or (vi) the *Acetobacter lovaniensis is Acetobacter lovaniensis* DSMZ 4491.

4. The use of claim 1 or 3, or the method of claim 2 or 3, wherein the composition further comprises at least one, two, three, four, or five microorganisms selected from the group consisting *Lactobacillus rhamnosus, Lactobacillus parafarraginis, Lactobacillus rapi, Lactobacillus zeae,* and *Lactobacillus harbinensis,* preferably the microbial composition further comprises *Lactobacillus rhamnosus, Lactobacillus parafarraginis, Lactobacillus rapi, Lactobacillus zeae,* and *Lactobacillus harbinensis.*

5. The use of any one of claims 1, 3 or 4, or the method of any one of claims 2-4, wherein the composition further comprises at least one, two, three, or four microorganisms selected from the group consisting of *Bacillus subtilis, Bacillus amyloliquefaciens, Bacillus licheniformis,* and *Bacillus megaterium,* preferably the microbial composition further comprises *Bacillus subtilis, Bacillus amyloliquefaciens, Bacillus licheniformis,* and *Bacillus megaterium.*

6. The use of any one of claims 1, 3, 4, or 5, or the method of claims 2-5, wherein the composition further comprises at least one, two, three, four, five, six, seven, eight, or nine microorganisms selected from the group consisting of *Lactobacillus buchneri, Lactobacillus diolivorans, Acetobacter indonesiensis, Pediococcus acidilactici, Acetobacter estunensis, Lactobacillus parabuchneri, Pseudomonas putida, Lactobacillus perolens,* and *Azotobacter vinelandii,* preferably the microbial composition further comprising *Lactobacillus buchneri, Lactobacillus diolivorans, Acetobacter indonesiensis, Pediococcus acidilactici, Acetobacter estunensis, Lactobacillus parabuchneri, Pseudomonas putida, Lactobacillus perolens,* and *Azotobacter vinelandii.*

7. The use of any one of claims 1, 3, 4, 5, or 6 or the method of any one of claims 2-6, wherein the plant is a pasture plant, an ornamental plant, or a crop plant, including a fruit, a vegetable, a nut, a seed or a grain.

8. A microbial composition comprising *Lactobacillus casei* DSMZ 20011, *Lactobacillus plantarum* DSMZ 21074, *Lactobacillus fermentum* DSMZ 20052, *Saccharomyces cerevisiae* DSMZ 70449, *Rhodopseudomonas palustris* ATCC 17001 and *Acetobacter lovaniensis* DSMZ 4491.

## Patentansprüche

1. Verwendung einer mikrobiellen Zusammensetzung, die *Lactobacillus casei, Lactobacillus plantarum, Lactobacillus fermentum, Saccharomyces cerevisiae, Rhodopseudomonas palustris* und *Acetobacter lovaniensis* umfasst, zur Behandlung oder Verhinderung einer Infektion einer Pflanze mit *Fusarium poae.*

2. Verfahren zur Behandlung oder Verhinderung einer Infektion einer Pflanze mit einem Pflanzenpathogen, wobei das Verfahren die Verabreichung einer wirksamen Menge einer mikrobiellen Zusammensetzung umfasst, die *Lactobacillus casei, Lactobacillus plantarum, Lactobacillus fermentum, Saccharomyces cerevisiae, Rhodopseudomonas palustris* und *Acetobacter lovaniensis* umfasst, an die Pflanze, den Samen der Pflanze, den Boden, in dem die Pflanze wurzelt, oder das anderweitige Exponieren der Pflanze oder des Samens der Pflanze, mit einer wirksamen Menge der mikrobiellen Zusammensetzung umfasst, wodurch die Pflanze behandelt oder eine Infektion der Pflanze verhindert wird, wobei die Infektion eine Infektion mit *F*. *poae* ist.

3. Verwendung nach Anspruch 1 oder Verfahren nach Anspruch 2, wobei (i) das *Lactobacillus casei Lactobacillus casei* DSMZ 20011 ist, und/oder (ii) das *Lactobacillus plantarum Lactobacillus plantarum* DSMZ 21074 ist, und/oder (iii) das *Lactobacillus fermentum Lactobacillus fermentum* DSMZ 20052 ist, und/oder (iv) der *Saccharomyces cerevisiae Saccharomyces cerevisiae* DSMZ 70449 ist, und/oder (v) der *Rhodopseudomonas palustris Rhodopseudomonas palustris* ATCC 17001 ist, und/oder (vi) der *Acetobacter lovaniensis Acetobacter lovaniensis* DSMZ 4491 ist.

4. Die Verwendung nach Anspruch 1 oder 3 oder das Verfahren nach Anspruch 2 oder 3, wobei die Zusammensetzung weiterhin mindestens einen, zwei, drei, vier oder fünf Mikroorganismen umfasst, ausgewählt aus der Gruppe bestehend aus *Lactobacillus rhamnosus, Lactobacillus parafarraginis, Lactobacillus rapi, Lactobacillus zeae* und *Lactobacillus harbinensis,* wobei die mikrobielle Zusammensetzung vorzugsweise weiterhin *Lactobacillus rhamnosus, Lactobacillus parafarraginis, Lactobacillus rapi, Lactobacillus zeae* und *Lactobacillus harbinensis* umfasst.

5. Die Verwendung nach einem der Ansprüche 1, 3 oder 4 oder das Verfahren nach einem der Ansprüche 2 bis 4, wobei die Zusammensetzung weiterhin mindestens einen, zwei, drei oder vier Mikroorganismen umfasst, ausgewählt aus der Gruppe bestehend aus *Bacillus subtilis, Bacillus amyloliquefaciens, Bacillus licheniformis,* und *Bacillus megaterium,* wobei die mikrobielle Zusammensetzung vorzugsweise weiterhin *Bacillus subtilis, Bacillus amyloliquefaciens, Bacillus licheniformis* und *Bacillus megaterium* umfasst.

6. Verwendung nach einem der Ansprüche 1, 3, 4 oder 5 oder das Verfahren nach einem der Ansprüche 2 bis 5, wobei die Zusammensetzung weiterhin mindestens einen, zwei, drei, vier, fünf, sechs, sieben, acht oder neun Mikroorganismen umfasst, ausgewählt aus der Gruppe bestehend aus *Lactobacillus buchneri, Lactobacillus diolivorans, Acetobacter indonesiensis, Pediococcus acidilactici, Acetobacter estunensis, Lactobacillus parabuchneri, Pseudomonas putida, Lactobacillus perolens,* und *Azotobacter vinelandii,* wobei die mikrobielle Zusammensetzung vorzugsweise weiterhin *Lactobacillus buchneri, Lactobacillus diolivorans, Acetobacter indonesiensis, Pediococcus acidilactici, Acetobacter estunensis, Lactobacillus parabuchneri, Pseudomonas putida, Lactobacillus perolens,* und *Azotobacter vinelandii* umfasst.

7. Verwendung nach einem der Ansprüche 1, 3, 4, 5 oder 6 oder das Verfahren nach einem der Ansprüche 2 bis 6, wobei die Pflanze eine Weidepflanze, eine Zierpflanze oder eine Kulturpflanze, einschließlich einer Frucht, eines Gemüses, einer Nuss, eines Samens oder eines Getreides ist.

8. Mikrobielle Zusammensetzung, umfassend *Lactobacillus casei* DSMZ 20011, *Lactobacillus plantarum* DSMZ 21074, *Lactobacillus fermentum* DSMZ 20052, *Saccharomyces cerevisiae* DSMZ 70449, *Rhodopseudomonas palustris* ATCC 17001 und *Acetobacter lovaniensis* DSMZ 4491.

## Revendications

1. Utilisation d'une composition microbienne comprenant *Lactobacillus casei, Lactobacillus plantarum, Lactobacillus fermentum, Saccharomyces cerevisiae, Rhodopseudomonas palustris* et *Acetobacter lovaniensis* pour le traitement ou la prévention d'une infection d'une plante par *Fusarium poae.*

2. Procédé de traitement ou de prévention d'une infection d'une plante avec un pathogène végétal, le procédé comprenant l'administration à la plante, la graine de la plante, le sol où la plante est enracinée, ou sinon d'exposition de la plante, ou de la graine de la plante à une quantité efficace d'une composition microbienne comprenant *Lactobacillus casei, Lactobacillus plantarum, Lactobacillus fermentum, Saccharomyces cerevisiae, Rhodopseudomonas palustris* et *Acetobacter lovaniensis,* traitant ainsi la plante ou empêchant une infection de la plante, dans lequel l'infection est une infection par F. *poae.*

3. Utilisation selon la revendication 1, ou procédé selon la revendication 2, dans laquelle·lequel (i) le *Lactobacillus casei* est *Lactobacillus casei* DSMZ 20011, et/ou (ii) le *Lactobacillus plantarum* est *Lactobacillus plantarum* DSMZ 21074, et/ou (iii) le *Lactobacillus fermentum* est *Lactobacillus fermentum* DSMZ 20052, et/ou (iv) le *Saccharomyces cerevisiae* est *Saccharomyces cerevisiae* DSMZ 70449, et/ou (v) le *Rhodopseudomonas palustris* est *Rhodopseudomonas palustris* ATCC 17001, et/ou (vi) *l'Acetobacter lovaniensis* est *Acetobacter lovaniensis* DSMZ 4491.

4. Utilisation selon la revendication 1 ou 3, ou procédé selon la revendication 2 ou 3, dans laquelle·lequel la composition comprend en outre au moins un, deux, trois, quatre, ou cinq micro-organismes sélectionnés dans le groupe constitué de *Lactobacillus rhamnosus, Lactobacillus parafarraginis, Lactobacillus rapi, Lactobacillus zeae,* et *Lactobacillus harbinensis,* préférablement la composition microbienne comprend en outre *Lactobacillus rhamnosus, Lactobacillus parafarraginis, Lactobacillus rapi, Lactobacillus zeae,* et *Lactobacillus harbinensis.*

5. Utilisation selon l'une quelconque des revendications 1, 3 ou 4, ou procédé selon l'une quelconque des revendications 2 à 4, dans laquelle·lequel la composition comprend en outre au moins un, deux, trois, ou quatre micro-organismes sélectionnés dans le groupe constitué de *Bacillus subtilis, Bacillus amyloliquefaciens, Bacillus licheniformis,* et *Bacillus megaterium,* préférablement la composition microbienne comprend en outre *Bacillus subtilis, Bacillus amyloliquefaciens, Bacillus licheniformis,* et *Bacillus megaterium.*

6. Utilisation selon l'une quelconque des revendications 1, 3, 4, ou 5, ou procédé selon les revendications 2 à 5, dans laquelle·lequel la composition comprend en outre au moins un, deux, trois, quatre, cinq, six, sept, huit, ou neuf micro-organismes sélectionnés dans le groupe constitué de *Lactobacillus buchneri, Lactobacillus diolivorans, Acetobacter indonesiensis, Pediococcus acidilactici, Acetobacter estunensis, Lactobacillus parabuchneri, Pseudomonas putida, Lactobacillus perolens,* et *Azotobacter vinelandii,* préférablement la composition microbienne comprenant en outre *Lactobacillus buchneri, Lactobacillus diolivorans, Acetobacter indonesiensis, Pediococcus acidilactici, Acetobacter estunensis, Lactobacillus parabuchneri, Pseudomonas putida, Lactobacillus perolens,* et *Azotobacter vinelandii.*

7. Utilisation selon l'une quelconque des revendications 1, 3, 4, 5, ou 6 ou procédé selon l'une quelconque des revendications 2 à 6, dans laquelle·lequel la plante est une plante de pâturage, une plante ornementale, ou une plante de grande culture, incluant un fruit, un légume, une noix, une graine ou une céréale.

8. Composition microbienne comprenant *Lactobacillus casei* DSMZ 20011, *Lactobacillus plantarum* DSMZ 21074, *Lactobacillus fermentum* DSMZ 20052, *Saccharomyces cerevisiae* DSMZ 70449, *Rhodopseudomonas palustris* ATCC 17001 et *Acetobacter lovaniensis* DSMZ 4491.
